# EUROPÄISCHE PATENTSCHRIFT

(11) **EP 2 686 026 B1**
(45) Veröffentlichungstag und Bekanntmachung des Hinweises auf die Patenterteilung: **13.03.2019**
(21) Anmeldenummer: 12709292.2
(22) Anmeldetag: 16.03.2012
(51) Int. Cl.: A61L 27/00, B01J 2/00, C09C 1/00, C09C 1/02, C08J 3/12, C08J 3/20, C08L 67/04

(54) **MIKROSTRUKTURIERTE VERBUNDTEILCHEN**
MICROSTRUCTURED COMPOSITE PARTICLES
PARTICULES COMPOSITES MICROSTRUCTURÉES

(30) Priorität: 18.03.2011 EP 11002245
(43) Veröffentlichungstag der Anmeldung: 22.01.2014
(73) Patentinhaber: Schaefer Kalk GmbH & Co. KG, 65582 Diez (DE)
(72) Erfinder: VUCAK, Marijan, 65624 Altendiez (DE); NOVER, Christoph, 47495 Rheinberg (DE)
(74) Vertreter: Mai Dörr Besier European Patent Attorneys
(86) Internationale Anmeldenummer: PCT/EP2012/001197
(87) Internationale Veröffentlichungsnummer: WO 2012/126600

(56) Entgegenhaltungen:
- EP-A1- 2 163 569
- US-A- 4 915 884
- US-A- 5 011 862
- US-A1- 2003 124 242
- US-B1- 6 403 219

## Beschreibung

Die vorliegende Erfindung betrifft mikrostrukturierte Verbundteilchen und ihre Verwendung.

Verbundwerkstoffe (Kompositwerkstoffe) sind bereits bekannt und bezeichnen einen Werkstoff aus zwei oder mehr verbundenen Materialien, der andere Werkstoffeigenschaften als seine einzelnen Komponenten besitzt. Für die Eigenschaften der Verbundwerkstoffe sind die stofflichen Eigenschaften und die Geometrie der Komponenten von Bedeutung. Insbesondere spielen oft Größeneffekte eine Rolle. Die Verbindung erfolgt in der Regel durch Stoff- oder Formschluss oder eine Kombination von beidem.

Auch mikrostrukturierte Verbundteilchen sind an sich schon bekannt.

Beispielsweise offenbart die europäische Patentanmeldung EP 0 523 372 A1 unter Verweis auf die japanische Patentanmeldung JP62083029 A ein Verfahren, bei welchem ein erstes Material (sogenannte Mutterpartikel) mit einem zweiten Material, das aus kleineren Partikeln (sogenannte Babypartikel) besteht, auf der Oberfläche beschichtet wird. Zu diesem Zweck wird vorzugsweise eine Oberflächenmodifizierungsvorrichtung ("*Hybridizer*") eingesetzt, die einen Hochgeschwindigkeitsrotor, einen Stator und ein kugelförmiges Gefäß, vorzugsweise aufweisend innenliegende Messer, umfasst.

Die Mutterpartikel und die Babypartikel werden gemischt, vorzugsweise feinstverteilt und in den Hybridizer eingebracht. Dort wird die Mischung vorzugsweise weiter feinstverteilt und vorzugsweise wiederholt mechanischen Kräften ausgesetzt, insbesondere Stoßkräften, Kompressionskräften, Reibungskräften und Scherkräften sowie den gegenseitigen Wechselwirkungen der Teilchen, um die Babyteilchen in den Mutterteilchen einheitlich einzubetten. Bevorzugte Rotorgeschwindigkeiten sollen im Bereich von 50 m/s bis 100 m/s, bezogen auf die Umfangsgeschwindigkeit, liegen.

Weiterhin beschreibt die europäische Patentanmeldung EP 0 523 372 A1 eine Fixierung der Verbundteilchen durch thermisches Plasmaspritzen, wobei vorzugsweise eine Unterdruckplasmasprühvorrichtung ("*reduced pressure plasma spraying device*") eingesetzt wird, die vorzugsweise eine Leistung von mindestens 30 kW aufweist.

Auf diese Weise wird ein medizinisches Material erhalten, das ein Substrat eines Materials hoher Festigkeit und hoher Biostabilität sowie eine darauf gebildete Schicht umfasst, die durch Plasmasprühen einer Substanz mit hoher Bioaffinität erhältlich ist. Bevorzugte Substanzen mit hoher Bioaffinität umfassen Hydroxylapatit, Tricalciumphosphat, Biogläser und andere Substanzen mit ähnlichen Eigenschaften.

Die deutsche Patentanmeldung DE 42 44 254 A1 betrifft ein Verfahren zur Herstellung von Verbundteilchen durch Befestigen einer Substanz auf der Oberfläche eines thermoplastischen Materials, das einen durchschnittlichen Teilchendurchmesser von 100 µm bis 10 mm hat, wobei die Substanz einen geringeren Teilchendurchmesser und eine bessere Hitzebeständigkeit hat als das thermoplastische Material, wobei das Verfahren die Schritte umfasst:
o zuerst Erhitzen der Substanz, die den geringeren Teilchendurchmesser und die bessere Hitzebeständigkeit hat als das thermoplastische Material, auf eine Temperatur, die nicht kleiner ist als der Erweichungspunkt des thermoplastischen Materials, unter Rühren in einer Vorrichtung, die ein Rührwerk und eine Heizeinrichtung hat;
o Zugeben des thermoplastischen Materials in die Vorrichtung; und
o Befestigen der Substanz mit der besseren Hitzebeständigkeit auf der Oberfläche des thermoplastischen Materials.

Als thermoplastisches Material (Mutterteilchen) der Verbundteilchen werden beispielsweise Pellets oder Perlen von verschiedenen kommerziell erhältlichen thermoplastischen Harzen oder Kunststoffe genannt, wie z. B. ABS, AS, MBS, Polyvinylchlorid, Polyacetal, Polyamid, Polyethylen, Polypropylen, Polyethylenterephthalat, Polystyrol, Polycarbonat, Polyacrylate. Der durchschnittliche Teilchendurchmesser der Mutterteilchen soll vorzugsweise im Bereich von 100 µm bis 10 mm sein.

Als Substanz (Babyteilchen) werden insbesondere Teilchen aus anorganischem Material, wie silikathaltige Teilchen (z. B. Glasperlen u. ä.), Aluminiumoxidteilchen und Zirkoniumoxidteilchen sowie Teilchen aus verschiedenen Metallen genannt, die ausgezeichnet wärmeresistent und abriebsresistent sind. Der durchschnittliche Teilchendurchmesser der Babyteilchen soll kleiner als der der Mutterteilchen sein und vorzugsweise 1/10 oder weniger des durchschnittlichen Teilchendurchmessers der Mutterteilchen, d. h. üblicherweise im Bereich von 1 µm bis 1 mm, sein.

Die europäische Patentanmeldung EP 0 922 488 A2 betrifft feste Verbundteilchen, die durch Modifikation der Oberfläche eines festen Partikels erhalten werden, indem man feine Partikel an der Oberfläche des Partikels befestigt, das als Kern fungiert, wobei man ein Kristall oder Kristalle der feinen Partikel auf einer Kombination des Kerns und der feinen Partikel wachsen lässt, um die feinen Partikel auf dem Kernpartikel in einer abschälfesten Weise unveränderlich zu fixieren.

Geeignete Kerne umfassen u. a. Cellulosederivate, Stärkederivate sowie synthetische Polymere, wie Nylon, Polyethylen und Polystyrol.

Geeignete feine Partikel umfassen u. a. Carbonate, Phosphate und Hydrogenphosphate, wie Calciummonohydrogenphosphat.

Das amerikanische Patent US 6,403,219 B1 offenbart feste Verbundteilchen, die durch Modifikation der Oberfläche eines festen Kernpartikels erhalten werden, indem man Partikel an der Oberfläche des Kernpartikels in nicht-abschälbarer Weise befestigt, wobei man ein Kristall einer festgelegten Partikelkomponente säulenförmig oder nadelförmig auf dem Kernpartikel wachsen lässt, wobei die festgelegten Partikel auf der Oberfläche des Kernpartikels angeklebt sind.

Geeignete Kerne umfassen u. a. Cellulosederivate, Stärkederivate sowie synthetische Polymere, wie Nylon, Polyethylen und Polystyrol.

Geeignete feine Partikel umfassen u. a. Carbonate, Phosphate und Hydrogenphosphate, wie Calciummonohydrogenphosphat.

Das Arbeitsbeispiel dieser Druckschrift beschreibt die Herstellung von Verbundteilchen aus Nylon 12 mit einem mittleren Teilchendurchmesser von 50 µm und tafelförmigen Calciummonohydrogenphosphat mit einer mittleren Seitenlänge von ungefähr 50 µm, wobei das Mengenverhältnis 10:1 beträgt. Die Verbundteilchen werden unter Verwendung einer Oberflächenmodifizierungsvorrichtung (Nara Machinery Co. NHS-0) erhalten. Dabei werden ein Pulver aus Nylon 12 und Calciummonohydrogenphosphat zunächst mit der Oberflächenmodifizierungsvorrichtung behandelt und das resultierende Produkt dann in einer Suspension von Calciummonohydrogenphosphat suspendiert, um die Kristalle auf der Oberfläche wachsen zu lassen. Die Vorgehensweise des Patents US 6,403,219 B1 ist daher mit einem erheblichen Aufwand verbunden.

Die japanische Patentanmeldung JP 9239020 A beschreibt ein Implantatmaterial für Hartgewebe, welches Verbundteilchen umfasst, bei welchen Partikel aus einem Material mit Bioaffinität, wie z. B. Hydroxylapatit oder Tricalciumphosphat, mit einer Ummantelung aus einem Material mit hoher Härte, wie z. B. Zirkoniumdioxid oder Aluminiumoxid, versehen sind.

Die Patentanmeldung US 2003/0124242 A1 betrifft Kapseln mit einer Mineralbeschichtung und einem Kern, der eine polyhydroxylierte Verbindung umfasst.

Als Bestandteile der Mineralbeschichtung werden Erdalkalicarbonate oder basische Carbonate, basische Übergangsmetallcarbonate, Erdalkali- und Übergangsmetallsulfate, Erdalkaliborate, Erdalkalihalogenide und gefälltes Silika genannt. Bevorzugte Erdalkalimetalle sollen Magnesium und Calcium umfassen.

Als polyhydroxylierte Verbindung werden Polysaccharide, wie Threose, Erythrose, Arabinose, Xylose, Ribose, Desoxyribose, Rhamnose, Fucose, Glucosamin, Galactosamin, N-Acetylglucosamin, N-Acetylgalactosamin, Stärke, Amylopektin, Amylose, Araban, Alginate, Carrageenan, Cellulose, Chitosan, Chondroitinsulfat, Dextran, Dextrin, Fructosan, Galaktan, Mannan, Gummiarabicum, Pektin, Ghattigummi, Galactosid, Glykan, Glycogen, Hemicellulose, Hyaluronsäure, Inulin, Lamarinarin, Levan, Mucoitinsulfat, Nigeran, Pentosan, Polydextrose und Xylan genannt.

Die Beispiele dieser Druckschrift beschreiben die Herstellung von Verbundteilchen aus Guar mit einer mittleren Teilchengröße von 32 µm und Hydroxylapatit mit einer mittleren Teilchengröße von 1,5 µm oder aus Stärke mit einer mittleren Teilchengröße von 10 µm und Calciumphosphat mit einer mittleren Teilchengröße von 1 µm. Die Verbundteilchen werden jeweils unter Verwendung einer Oberflächenmodifizierungsvorrichtung (Nara Machinery Co. NHS-0) erhalten.

Das Patent US 5,011,862 beschreibt thermoplastische Hohlkugeln, beispielsweise aus PVC, PAN, Polyalkylmethacrylat, PS, die an ihrer Oberfläche befestigte oder eingebaute Trübungsmittel, wie TiO₂, ZnO, CaCO₃, Talk, Tonmaterialien o. ä. umfassen.

Das Patent US 4,915,884 offenbart ein Granulat, welches durch homogenes Mischen eines thermoplastischen Harzes, wie z. B. PE, und eines Zusatzstoffes, wie z. B. Calciumcarbonat, anschließendes Extrudieren dieser Mischung und Zerkleinerung des resultierenden Extrusionstrangs erhalten wird.

Die europäische Patentanmeldung EP 2163 569 A1 betrifft ein Verfahren zur Herstellung von Harzteilchen aus einem saure Gruppen enthaltenden, thermoplastischen Harz oder Elastomer und einem Füllstoff, wie z. B. Calciumcarbonat. Das Verfahren erzeugt Harzteilchen durch Schmelzen und Mischen eines saure Gruppen enthaltenden, thermoplastischen Harzes oder Elastomers mit Füllstoff-Teilchen und einem wasserlöslichen Material, um eine Harzzusammensetzung mit feinen Harzteilchen bereitzustellen, die durch das thermoplastische Harz und die Füllstoff-Teilchen gebildet werden. Die Harzzusammensetzung wird in einer Matrix dispergiert, die das wasserlösliche Material umfasst. Anschließend wird die Matrix-Komponente aus der Harzzusammensetzung wieder entfernt, um die Harzteilchen zu liefern. Die resultierenden Harzteilchen umfassen jeweils ein Kernteilchen, das das saure Gruppe enthaltende thermoplastische Harz oder Elastomer umfasst, und Füllstoff-Teilchen, die auf der Außenseite des Kernteilchens immobilisiert sind. Dabei führt die spezielle, äußerst aufwendige Vorgehensweise dieser Druckschrift zu einer porösen Struktur der Harzteilchen.

Die Druckschriften Y. Shi, Y. Sun Fabrication and Characterization of a Novel Biporous Spherical Adsorbent for Protein Chromatography Chromatographia 2003, 57, S. 29-35 und L. Wu, S. Bai und Y. Sun Development of Rigid Bidisperse Porous Microspheres for High-Speed Protein Chromatography Biotechnol. Prog. 2003, 19, S. 1300-1306 beschreiben die Herstellung von Polymerperlen durch Suspensionspolymerisation von Polyglycidylmethacrylat-Copolymeren in Gegenwart von superfeinem Calciumcarbonat. Nach der Polymerisation wird das Calciumcarbonat herausgewaschen, um Polymerperlen mit porösen Strukturen zu erhalten.

Keine der vorstehend genannten Druckschriften zieht jedoch die Verwendung von gefällten Calciumcarbonat-Teilchen (PCC) oder von resorbierbaren Polyestern als Bestandteil der beschriebenen Verbundteilchen in Betracht.

Weiterhin ist zwar auch der Einsatz von gefälltem Calciumcarbonat in Verbindung mit Compounds schon literaturbekannt, aber wiederum nicht als Bestandteil von Verbundteilchen.

So beschreibt die Veröffentlichung T. D. Lam, T. V. Hoang, D. T. Quang, J. S. Kim Effect of nanosized and surface-modified precipitated calcium carbonate on properties of CaCO3/polypropylene nanocomposites Materials Science and Engineering A 501 (2009) 87-93 den Effekt von oberflächenmodifizierten Calciumcarbonat-Nanopartikeln auf die Eigenschaften von CaCO₃/Polypropylen-Nanokompositen. Dabei werden die Calciumcarbonat-Teilchen jedoch als Füllstoff im Polymer homogen dispergiert.

Die Publikation L. Jiang, Y. C. Lam, K. C. Tam, D. T. Li, J. Zhang The influence of fatty acid coating on the rheological and mechanical properties of thermoplastic polyurethane (TPU)/nano-sized precipitated calcium carbonate (NPCC) composites Polymer Bulletin 57, 575-586 (2006) beschäftigt sich mit dem Einfluss von Fettsäurebeschichtungen auf die rheologischen und mechanischen Eigenschaften von Kompositen, umfassend thermoplastisches Polyurethan und gefällte Calciumcarbonat-Nanopartikel. Auch hier werden die Calciumcarbonat-Teilchen als Füllstoff im Polymer homogen dispergiert.

Die Abhandlung J. Cayer-Barrioz, L. Ferry, D. Frihi, K. Cavalier, R. Séguéla, G. Vigier Microstructure and Mechanical Behavior of Polyamide 66-Precipitated Calcium Carbonate Composites: Influence of the Particle Surface Treatment Journal of Applied Polymer Science, Vol. 100, 989-999 (2006) setzt sich mit der Mikrostruktur und dem mechanischen Verhalten von Kompositen auseinander, die Polyamid 66 und gefällte Calciumcarbonat-Nanopartikel umfassen. Wiederum werden die Calciumcarbonat-Teilchen als Füllstoff im Polymer homogen dispergiert.

Die japanische Patentanmeldung JP 41 39020 A beschreibt die Polymerisation von polybasischen Carbonsäuren oder polyfunktionellen Isocyanaten in einer Calciumcarbonat-Zusammensetzung, die kolloidales oder partikelförmiges Calciumcarbonat umfasst. Auch hier werden die Calciumcarbonat-Teilchen als Füllstoff im Polymer homogen dispergiert.

Die Publikation M. Avella, S. Cosco, M. L. Di Lorenzo, E. Di Pace, M. E. Errico Influence of CaCO3 Nanoparticles Shape on Thermal and Crystallization Behavior of Isotactic Polypropylene based Nanocomposites Journal of Thermal Analysis and Calorimetry, Vol. 80 (2005) 131-136 beschreibt den Einfluss der Gestalt von CaCO₃ Nanopartikeln auf das thermische Verhalten und das Kristallisationsverhalten von auf isotaktischen Polypropylen-basierenden Nanokompositen. Dabei werden die Calciumcarbonat-Teilchen als Füllstoff im Polymer homogen dispergiert.

Die Publikation S. Weihe, M. Wehmöller, C. Schiller, C. Rasche, H. Eufinger, M. Epple Formgebung degradierbarer Werkstoffe mit Hilfe der Verfahrenskette zur Fertigung individueller CAD/CAM-Implantate Biomedizinische Technik/Biomedical Engineering, Band 46, Heft s1, Seiten 214-215 offenbart eine Implantatherstellung mittels Schmelzpressen von Polyglycolid-co-lactid (PGLA), Poly-L-Iactid (PLLA) und gradierten Werkstoffen aus mehreren Polymeren und Pressen unter Verwendung einer Edelstahlform.

Weiterhin wird ein Begasungsverfahren unter Verwendung von Poly-DL-Iactid (PDLLA) und einer Teflonform beschrieben. Dieses beruht auf einer Begasung mit CO₂ unter hohem Druck bei Raumtemperatur. Dabei wird CO₂ vom Polymer aufgenommen und dessen Glasübergangstemperatur gesenkt. Bei Druckreduktion kommt es zum Aufschäumen des amorphen PDLLA und exaktem Ausfließen der Form. Das Verfahren soll damit das Einbringen thermolabiler Substanzen, z. B. Antibiotika und osteoinduktiver Proteine, ermöglichen und auch für andere amorphe Polymere, wie z. B. PGLA, anwendbar sein.

Ein Nachteil von herkömmlichen Polylactidimplantatmaterialien ist darin zu sehen, dass sie im Röntgenbild nicht sichtbar sind. Eine Überprüfung des Behandlungsfortschritts durch Röntgen ist daher nicht möglich.

Vor diesem Hintergrund lag der vorliegenden Erfindung die Aufgabe zugrunde, eine möglichst effiziente und preiswerte Lösung insbesondere für die folgenden Problemstellungen anzugeben:
Zum einen wurde nach Möglichkeiten gesucht, um die Eigenschaften von Polymeren, bevorzugt von thermoplastisch verarbeitbaren Polymeren, besonders bevorzugt von Polyamiden und von resorbierbaren Polymeren, vorzugsweise von resorbierbaren Polyestern, insbesondere von Poly-D-, Poly-L- und/oder Poly-D,L-milchsäure, bevorzugt hinsichtlich ihrer mechanischen Eigenschaften und/oder ihrer Eignung für medizintechnische Anwendungen, gezielt zu verbessern.

Weiterhin sollten Möglichkeiten aufgezeigt werden, wie Calciumsalze, insbesondere Calciumcarbonat, möglichst homogen in einem Polymer, bevorzugt in einem thermoplastisch verarbeitbaren Polymer, besonders bevorzugt in einem Polyamid und/oder in einem resorbierbaren Polymer, vorzugsweise in einem resorbierbaren Polyester, insbesondere in Poly-D-, Poly-L- und/oder Poly-D,L-milchsäure, dispergiert werden können.

Dabei sollte die erfindungsgemäße Lösung auf möglichst einfache Art und Weise, möglichst kostengünstig realisiert werden können.

Weiterhin war eine Weiterverarbeitbarkeit der resultierenden Polymere auf möglichst einfache Art und Weise gewünscht.

Besonders im Vordergrund stand der Wunsch nach möglichst guten mechanischen Eigenschaften sowie einer möglichst guten pH-Wert-Stabilisierung, die insbesondere für Anwendungen im medizintechnischen Bereich von Vorteil sind.

Darüber hinaus wurden Materialien, insbesondere für medizintechnische Anwendungen, angestrebt, die eine möglichst gute Resorbierbarkeit aufweisen. Besonders gewünscht wurde eine Lösung für das Problem, dass resorbierbare Polyester, insbesondere Polymilchsäure für Lasersinter-Anwendungen nur bedingt geeignet ist, da sie nur sehr schwer, falls überhaupt, auf Teilchengrößen kleiner 150 µm, zweckmäßigerweise kleiner 125 µm, insbesondere im Bereich von 50 µm bis 70 µm, gemahlen werden können. Wünschenswert waren in diesem Zusammenhang Lösungen, die ein Stauben des gemahlenen Produkts möglichst verhindern und insbesondere seine Verwendung auch für die Herstellung von Mikrobauteilen und seine Verwendung in Reinräumen erlauben.

Weiterhin sollten auch die Eigenschaften von Polymerschäumen, insbesondere von solchen für medizintechnische Anwendungen, wie Implantate, verbessert werden.

Schließlich sollten auch Möglichkeiten aufgezeigt werden, wie bei Verwendung von Implantatmaterialien Behandlungsfortschritte auf möglichst einfache Art und Weise festgestellt und überprüft werden können.

Gelöst werden diese sowie weitere nicht konkret genannten Aufgaben, die sich aus den obigen Zusammenhängen direkt ableiten lassen, durch die Bereitstellung von mikrostrukturierten Verbundteilchen mit allen Merkmalen des vorliegenden Anspruch 1. Die auf Anspruch 1 rückbezogenen Unteransprüche beschreiben besonders zweckmäßige Varianten der Verbundteilchen. Weiterhin werden besonders zweckmäßige Anwendungen der erfindungsgemäßen Verbundteilchen unter Schutz gestellt.

Durch die Zugänglichmachung von mikrostrukturierten Verbundteilchen, die durch ein Verfahren erhältlich sind, bei welchem man große Teilchen mit kleinen Teilchen verbindet, wobei
- die großen Teilchen einen mittleren Teilchendurchmesser im Bereich von 0,1 µm bis 10 mm aufweisen,
- der mittlere Teilchendurchmesser der kleinen Teilchen höchstens 1/10 des mittleren Teilchendurchmessers der großen Teilchen ist,
- die großen Teilchen mindestens ein Polymer umfassen,
- die kleinen Teilchen Calciumcarbonat umfassen,
- die kleinen Teilchen auf der Oberfläche der großen Teilchen angeordnet und/oder inhomogen innerhalb der großen Teilchen verteilt sind,
- die kleinen Teilchen gefällte Calciumcarbonat-Teilchen mit einer mittleren Teilchengröße im Bereich von 0,01 µm bis 1,0 mm umfassen, gelingt es auf nicht ohne weiteres vorhersehbare Weise, die vorstehend genannten Probleme auf äußerst effiziente und preiswerte Art und Weise zu lösen.

Zum einen ist es möglich, durch Zugabe der erfindungsgemäßen Verbundteilchen als Additiv zu thermoplastisch verarbeitbaren Polymeren, vor allem zu thermoplastischen Polymeren, bevorzugt zu Polyamiden und zu resorbierbaren Polymeren, vorzugsweise zu resorbierbaren Polyestern, insbesondere zu Poly-D-, Poly-L- und/oder Poly-D,L-milchsäure, deren Eigenschaften, vor allem hinsichtlich ihrer mechanischen Eigenschaften sowie ihrer Eignung für medizintechnische Anwendungen, gezielt zu verbessern.

Weiterhin kann auf diese Weise Calciumcarbonat äußerst homogen in einem Polymer, bevorzugt in einem thermoplastisch verarbeitbaren Polymer, besonders bevorzugt in einem Polyamid und/oder in einem resorbierbaren Polymer, vorzugsweise in einem resorbierbaren Polyester, insbesondere in Poly-D-, Poly-L- und/oder Poly-D,L-milchsäure, dispergiert werden.

Dabei können die resultierenden Polymere auf vergleichbar einfache Art und Weise weiterverarbeitet werden.

Vergleicht man die Einzelteilchen untereinander, so sind die erfindungsgemäßen Verbundteilchen sehr einheitlich und zeichnen sich durch eine sehr hohe Homogenität, sowohl hinsichtlich ihrer Zusammensetzung, als auch hinsichtlich ihrer Struktur aus.

Weiterhin weisen die erfindungsgemäßen Verbundteilchen, sowohl als Einzelkomponente, als auch als Additiv in einem Matrixpolymer, jeweils verglichen mit dem reinen thermoplastischen Polymer oder Polymergemisch, in der Regel verbesserte mechanische Eigenschaften sowie eine verbesserte pH-Wert-Stabilisierung auf. Diese Eigenschaften machen sie insbesondere für Anwendungen im medizintechnischen Bereich interessant.

Darüber hinaus weisen die erfindungsgemäßen Verbundteilchen eine sehr gute Resorbierbarkeit, insbesondere im menschlichen Organismus, auf.

Die erfindungsgemäßen Verbundteilchen können auf vergleichweise einfache Art und Weise gemahlen werden. Die Staubeinwicklung hierbei ist äußerst gering, da beim Vermahlen ein Verkleben der Polymerpartikel durch das Calciucarbonat reduziert und ein besseres Mahlergebnis erzielt wird. Weiterhin wird eine lokale Überhitzung des Mahlgutes bestmöglich vermieden, so dass auch thermisch degradierbare Polymere sehr leicht verarbeitet werden können. Darüber hinaus wird eine separate Zugabe der Einzelkomponenten (Calciumsalz und Polymer) vermieden, welches ebenfalls die Staubbildung während der Verarbeitung deutlich reduziert. In der Summe ermöglicht diese Vorgehensweise daher die gezielte Einstellung der Teilchengröße der gemahlenen Teilchen, ihrer Fließeigenschaften, ihrer Rieselfähigkeit sowie ihrer Staubneigung und somit vor allem auch die Verwendung der gemahlenen Produkte in ultradünnen Flächen und in staubempfindlichen Anwendungen, insbesondere auch für die Herstellung von Mikrobauteilen und für die Verwendung in Reinräumen. Darüber hinaus ist auch bei den ungemahlenen Verbundteilchen keine Staubneigung zu beobachten. Die erfindungsgemäße Lösung eignet sich daher vor allem für die Herstellung von Polymilchsäure-Teilchen für Rapid Prototyping (additive Fertigungsverfahren), insbesondere für Lasersinter-Anwendungen, wobei die Polymilchsäure-Teilchen in diesem Zusammenhang vorzugsweise eine mittlere Teilchengrößen kleiner 150 µm, bevorzugt kleiner 125 µm, insbesondere im Bereich von 50 µm bis 70 µm, aufweisen.

Weiterhin werden auch die Eigenschaften von Polymerschäumen, insbesondere von solchen für medizintechnische Anwendungen, wie Implantate, deutlich verbessert.

Darüber hinaus ermöglicht die Verwendung der erfindungsgemäßen Verbundteilchen in Implantatmaterialien auch eine vergleichsweise einfache Überprüfung und Verifizierung des Behandlungsfortschritts, da die erfindungsgemäßen Verbundteilchen beim Röntgen sichtbar sind und somit das Implantat im Körper durch Röntgen direkt beobachtet werden kann.

Gegenstand der vorliegenden Erfindung sind dementsprechend mikrostrukturierte Verbundteilchen (Kompositteilchen), die durch ein Verfahren erhältlich sind, bei welchem man große Teilchen mit kleinen Teilchen verbindet.

Als Mikrostruktur werden in der vorliegenden Erfindung die mikroskopischen Eigenschaften eines Materials bezeichnet. Dazu gehören unter anderem die auflösbare Feinstruktur und das Gefüge. Bei Flüssigkeiten sowie Gasen sind diese nicht vorhanden. Hier befinden sich die einzelnen Atome oder Moleküle in einem ungeordneten Zustand. Amorphe Festkörper weisen zumeist eine strukturelle Nahordnung im Bereich der Nachbaratome auf, jedoch keine Fernordnung. Kristalline Festkörper hingegen weisen nicht nur im Nahbereich, sondern auch im Fernbereich eine geordnete Gitterstruktur auf.

Im Rahmen der vorliegenden Erfindung umfassen die großen Teilchen mindestens ein Polymer.

Bei den Verbundteilchen nach Anspruch 1 unterliegt dieses Polymer grundsätzlich keinen weiteren Beschränkungen. Es handelt sich jedoch bevorzugt um ein thermoplastisches Polymer, zweckmäßigerweise ein Biopolymer, ein Kautschuk, insbesondere Naturkautschuk oder Synthesekautschuk, und/oder ein Polyurethan.

Der Begriff "*thermoplastisches Polymer*" bezeichnet in diesem Zusammenhang einen Kunststoff, der sich in einem bestimmten Temperaturbereich, bevorzugt im Bereich von 25°C bis 350°C, (thermoplastisch) verformen lässt. Dieser Vorgang ist reversibel, das heißt er kann durch Abkühlung und Wiedererwärmung bis in den schmelzflüssigen Zustand beliebig oft wiederholt werden, solange nicht durch Überhitzung die sogenannte thermische Zersetzung des Materials einsetzt. Darin unterscheiden sich thermoplastische Polymere von den Duroplasten und Elastomeren.

Der Begriff "*Biopolymer*" bezeichnet einen Werkstoff, der aus biogenen Rohstoffen (nachwachsenden Rohstoffen) besteht und/oder biologisch abbaubar ist (biogenes und/oder biologisch abbaubares Polymer). Unter diesen Begriff fallen also biobasierte Biopolymere, die biologisch abbaubar oder auch nicht biologisch abbaubar sind, als auch erdölbasierte Polymere, die biologisch abbaubar sind. Damit erfolgt eine Abgrenzung von den konventionellen, erdölbasierten Werkstoffen bzw. Kunststoffen, die nicht biologisch abbaubar sind, wie z. B. Polyethylen (PE), Polypropylen (PP) und Polyvinylchlorid (PVC).

Der Begriff "*Kautschuk*" bezeichnet ein hochmolekulares, unvernetztes polymeres Material mit gummi-elastischen Eigenschaften bei Raumtemperatur (25°C). Bei höheren Temperaturen oder unter Einfluss von Verformungskräften zeigt ein Kautschuk ein zunehmendes viskoses Fließen und ermöglicht so sein Umformen unter geeigneten Bedingungen.

Gummi-elastisches Verhalten ist durch einen relativ geringen Schermodul mit einer eher geringen Temperaturabhängigkeit gekennzeichnet. Es wird durch Entropieänderungen verursacht. Durch Verstrecken wird das gummi-elastische Material in eine geordnetere Konfiguration gezwungen, die zu einer Entropieabnahme führt. Nach Entfernung der Kraft kehren die Polymere daher wieder in ihre Ursprungsposition zurück und die Entropie erhöht sich wieder.

Der Begriff "*Polyurethan*" (PU, DIN-Kurzzeichen: PUR) bezeichnet einen Kunststoff oder ein Kunstharz, welcher oder welches aus der Polyadditionsreaktion von Diolen oder Polyolen mit Polyisocyanaten entsteht. Charakteristisch für ein Polyurethan ist die Urethan-Gruppe.

Im Rahmen der vorliegenden Erfindung werden thermoplastische Polymere besonders bevorzugt eingesetzt. Besonders geeignete Polymere schließen dabei die folgenden Polymere ein: Acrylnitril-Ethylen-Propylen-(Dien)-Styrol-Copolymer, Acrylnitril-Methacrylat-Copolymer, Acrylnitril-Methylmethacrylat-Copolymer, Acrylnitril-chloriertes Polyethylen-Styrol-Copolymer, Acrylnitril-Butadien-Styrol-Copolymer, Acrylnitril-Ethylen-Propylen-Styrol-Copolymer, aromatische Polyester, Acrylnitril-Styrol-Acrylester-Copolymer, Butadien-Styrol-Copolymer, Celluloseacetat, Celluloseacetobutyrat, Celluloseacetopropionat, hydrierte Cellulose, Carboxymethylcellulose, Cellulosenitrat, Cellulosepropionat, Cellulosetriacetat, Polyvinylchlorid, Ethylen-Acrylsäure-Copolymer, Ethylen-Butylacrylat-Copolymer, Ethylen-Chlortrifluorethylen-Copolymer, Ethylen-Ethylacrylat-Copolymer, Ethylen-Methacryat-Copolymer, Ethylen-Methacrylsäure-Copolymer, Ethylen-Tetrafluorethylen-Copolymer, Ethylen-Vinylalkohol-Copolymer, Ethylen-Buten-Copolymer, Ethylcellulose, Polystyrol, Polyfluorethylenpropylen, Methylmethacrylat-Acrylnitril-Butadien-Styrol-Copolymer, Methylmethacrylat-Butadien-Styrol-Copolymer, Methylcellulose, Polyamid 11, Polyamid 12, Polyamid 46, Polyamid 6, Polyamid 6-3-T, Polyamid 6-Terephthalsäure-Copolymer, Polyamid 66, Polyamid 69, Polyamid 610, Polyamid 612, Polyamid 6I, Polyamid MXD 6, Polyamid PDA-T, Polyamid, Polyarylether, Polyaryletherketon, Polyamidimid, Polyarylamid, Polyaminobismaleinimid, Polyarylate, Polybuten-1, Polybutylacrylat, Polybenzimidazol, Polybismaleinimid, Polyoxadiazobenzimidazol, Polybutylenterephthalat, Polycarbonat, Polychlortrifluorethylen, Polyethylen, Polyestercarbonat, Polyaryletherketon, Polyetheretherketon, Polyetherimid, Polyetherketon, Polyethylenoxid, Polyarylethersulfon, Polyethylenterephthalat, Polyimid, Polyisobutylen, Polyisocyanurat, Polyimidsulfon, Polymethacrylimid, Polymethacrylat, Poly-4-methylpenten-1, Polyacetal, Polypropylen, Polyphenylenoxid, Polypropylenoxid, Polyphenylensulfid, Polyphenylensulfon, Polystyrol, Polysulfon, Polytetrafluroethylen, Polyurethan, Polyvinylacetat, Polyvinylalkohol, Polyvinylbutyral, Polyvinylchlorid, Polyvinylidenchlorid, Polyvinylidenfluorid, Polyvinylfluorid, Polyvinylmethylether, Polyvinylpyrrolidon, Styrol-Butadien-Copolymer, Styrol-Isopren-Copolymer, Styrol-Maleinsäureanhydrid-Copolymer, Styrol-Maleinsäureanhydrid-Butadien-Copolymer, Styrol-Methylmethacrylat-Copolymer, Styrol-Methylstyrol-Copolymer, Styrol-Acrylnitril-Copolymer, Vinylchlorid-Ethylen-Copolymer, Vinylchlorid-Methacrylat-Copolymer, Vinylchlorid-Maleinsäureanhydrid-Copolymer, Vinylchlorid-Maleinimid-Copolymer, Vinylchlorid-Methylmethacrylat-Copolymer, Vinylchlorid-Octylacrylat-Copolymer, Vinylchlorid-Vinylacetat-Copolymer, Vinylchlorid-Vinylidenchlorid-Copolymer und Vinylchlorid-Vinylidenchlorid-Acrylnitril-Copolymer.

Weiterhin ist auch die Verwendung von folgenden Kautschuken besonders vorteilhaft: natürlich vorkommendes Polyisopren, insbesondere cis-1,4-Polyisopren (Naturkautschuk; NR) und trans-1,4-Polyisopren (Guttapercha), vor allem Naturkautschuk; Nitrilkautschuk (Copolymer von Butadien und Acrylnitril; Poly(acrylnitril-co-1,3-butadien; NBR; so genannter Buna N-Kautschuk); Butadienkautschuk (Polybutadien; BR); Acrylkautschuk (Polyacrylkautschuk; ACM, ABR); Fluorkautschuk (FPM); Styrol-Butadien-Kautschuk (Copolymer von Styrol und Butadien; SBR); Styrol-Isopren-Butadien-Kautschuk (Copolymer von Styrol, Isopren und Butadien; SIBR); Polybutadien; synthetischer Isoprenkautschuk (Polyisopren; IR); Ethylen-Propylen-Kautschuk (Copolymer von Ethylen und Propylen; EPM); Ethylen-Propylen-Dien-Kautschuk (Terpolymer von Ethylen, Propylen und einer Dien-Komponente; EPDM); Butylkautschuk (Copolymer von Isobutylen und Isopren; IIR); Ethylen-Vinylacetat-Kautschuk (Copolymer von Ethylen und Vinylacetat; EVM); Ethylen-Methylacrylat-Kautschuk (Copolymer von Ethylen und Methylacrylat; AEM); Epoxidkautschuk, wie Polychlormethyloxiran (Epichlorhydrinpolymer; CO), Ethylenoxid (Oxiran) - Chlormethyloxiran (Epichlorhydrinpolymer; ECO), Epichlorhydrin - Ethylenoxid - Allylglycidyletherterpolymer (GECO), Epichlorhydrin - Allylglycidylethercopolymer (GCO) und Propylenoxid - Allylglycidylethercopolymer (GPO); Polynorbornen-Kautschuk (Polymer von Bicyclo[2.2.1]hept-2-en (2-Norbornen); PNR); Polyalkenylen (Polymer von Cycloolefinen); Siliconkautschuk (Q), wie Siliconkautschuk nur mit Methylsubstituenten an der Polymerkette (MQ; z. B. Dimethylpolysiloxan), Siliconkautschuk mit Methylvinyl- und Vinylsubstituentengruppen an der Polymerkette (VMQ), Siliconkautschuk mit Phenyl- und Methylsubstituenten an der Polymerkette (PMQ), Siliconkautschuk mit Fluor- und Methylgruppen an der Polymerkette (FMQ), Siliconkautschuk mit Fluor-, Methyl- und Vinylsubstituenten an der Polymerkette (FVMQ); Polyurethankautschuk; Thiolkautschuk; Halogenbutylkautschuk, wie Brombutylkautschuk (BIIR) und Chlorbutylkautschuk (CIIR); Chlorpolyethylen (CM); Chlorsulfonylpolyethylen (CSM); hydrierter Nitrilkautschuk (HNBR); und Polyphosphazen.

Besonders bevorzugte Nitrilkautschuke schließen statistische Terpolymere von Acrylnitril, Butadien und einer Carbonsäure, wie Methacrylsäure, ein. In diesem Zusammenhang umfasst der Nitrilkautschuk vorzugsweise, bezogen auf das Gesamtgewicht des Polymers, die folgenden Hauptkomponenten: 15,0 Gew.-% bis 42,0 Gew.-% Acrylnitrilpolymer; 1,0 Gew.-% bis 10,0 Gew.-% Carbonsäure und der Rest ist überwiegend Butadien (z. B. 38,0 Gew.-% bis 75,0 Gew.-%). Typischerweise ist die Zusammensetzung: 20,0 Gew.-% bis 40,0 Gew.-% Acrylnitrilpolymer, 3,0 Gew.-% bis 8,0 Gew.-% Carbonsäure und 40,0 Gew.-% bis 65,0 Gew.-% oder 67,0 Gew.-% sind Butadien. Besonders bevorzugte Nitrilkautschuke schließen ein Terpolymer von Acrylnitril, Butadien und einer Carbonsäure ein, bei welchem der Acrylnitrilgehalt kleiner als 35,0 Gew.-% ist und der Carbonsäuregehalt kleiner als 10,0 Gew.-% ist, wobei der Butadiengehalt dem verbleibenden Rest entspricht. Noch mehr bevorzugte Nitrilkautschuke können folgende Mengen umfassen: 20,0 Gew.-% bis 30,0 Gew.-% Acrylnitrilpolymer, 4,0 Gew.-% bis 6,0 Gew.-% Carbonsäure und der Rest ist überwiegend Butadien.

Der Einsatz von Stickstoff-haltigen Polymeren, insbesondere von Polyamiden, ist im Rahmen der vorliegenden Erfindung besonders günstig. Besonders bevorzugt werden Polyamid 11, Polyamid 12, Polyamid 46, Polyamid 6, Polyamid 6-3-T, Polyamid 6-Terephthalsäure-Copolymer, Polyamid 66, Polyamid 69, Polyamid 610, Polyamid 612, Polyamid 6I, Polyamid MXD 6 und/oder Polyamid PDA-T, insbesondere Polyamid 12.

Weiterhin ist auch die Verwendung von resorbierbaren Polymeren, insbesondere von resorbierbaren Polyestern, besonders zweckmäßig. Unter dem Begriff "Resorption" (lat. resorbere = "aufsaugen") versteht man die Stoffaufnahme in biologischen Systemen, insbesondere in den menschlichen Organismus. Vorliegend von Interesse sind insbesondere solche Materialien, die für die Herstellung von resorbierbaren Implantaten verwendet werden können.

Erfindungsgemäß besonders bevorzugte, resorbierbare Polymere umfassen Wiederholungseinheiten der Milchsäure, der Hydroxybuttersäure und/oder der Glycolsäure, bevorzugt der Milchsäure und/oder der Glycolsäure, insbesondere der Milchsäure. Polymilchsäuren werden dabei besonders bevorzugt. Darüber hinaus ist auch der Einsatz von Poly(dioxanon) besonders vorteilhaft.

Unter "Polymilchsäure" werden hier Polymere verstanden, die aus Milchsäureeinheiten aufgebaut sind. Solche Polymilchsäuren werden üblicherweise durch Kondensation von Milchsäuren hergestellt, werden aber auch bei der ringöffnenden Polymerisation von Lactiden unter geeigneten Bedingungen erhalten.

Erfindungsgemäß besonders geeignete, resorbierbare Polymere schließen Poly(glycolid-co-L-lactid), Poly(L-lactid), Poly(L-lactid-co-ε-caprolacton), Poly(L-lactid-co-glycolid), Poly(L-lactid -co-D,L-lactid), Poly(D,L-lactid-co-glycolid) sowie Poly(dioxanon) ein. Derartige Polymere sind beispielsweise von der Firma Boehringer Ingelheim Pharma KG (Deutschland) unter den Handelsnamen Resomer® GL 903, Resomer® L 206 S, Resomer® L 207 S, Resomer® L 209 S, Resomer® L 210, Resomer® L 210 S, Resomer® LC 703 S, Resomer® LG 824 S, Resomer® LG 855 S, Resomer® LG 857 S, Resomer® LR 704 S, Resomer® LR 706 S, Resomer® LR 708, Resomer® LR 927 S, Resomer® RG 509 S und Resomer® X 206 S kommerziell erhältlich.

Für die Zwecke der vorliegenden Erfindung besonders vorteilhafte, resorbierbare Polymere, bevorzugt handelt es sich hierbei um resorbierbare Polyester, vorzugsweise um Milchsäurepolymere, insbesondere um Poly-D-, Poly-L- oder Poly-D,L-milchsäuren, haben ein Zahlenmittel des Molekulargewichts (Mn), vorzugsweise bestimmt durch Gelpermeationschromatographie gegen engverteilte Polystyrol-Standards oder durch Endgruppentitration, größer 500 g/mol, bevorzugt größer 1.000 g/mol, besonders bevorzugt größer 5.000 g/mol, zweckmäßigerweise größer 10.000 g/mol, insbesondere größer 25.000 g/mol. Andererseits ist das Zahlenmittel bevorzugter resorbierbarer Polymere kleiner 1.000.000 g/mol, zweckmäßigerweise kleiner 500.000 g/mol, günstigerweise kleiner 100.000 g/mol, insbesondere höchstens 50.000 g/mol. Ein Zahlenmittel des Molekulargewichts im Bereich von 500 g/mol bis 50.000 g/mol hat sich im Rahmen der vorliegenden Erfindung ganz besonders bewährt.

Das Gewichtsmittel des Molekulargewichts (Mw) bevorzugter resorbierbarer Polymere, vorzugsweise handelt es sich hierbei um resorbierbare Polyester, günstigerweise um Milchsäurepolymere, insbesondere um Poly-D-, Poly-L- oder Poly-D,L-milchsäuren, vorzugsweise bestimmt durch Gelpermeationschromatographie gegen engverteilte Polystyrol-Standards, liegt vorzugsweise im Bereich von 750 g/mol bis 5.000.000 g/mol, bevorzugt im Bereich von 750 g/mol bis 1.000.000 g/mol, besonders bevorzugt im Bereich von 750 g/mol bis 500.000 g/mol, insbesondere im Bereich von 750 g/mol bis 250.000 g/mol, und die Polydispersität dieser Polymere ist günstigerweise im Bereich von 1,5 bis 5.

Die inhärente Viskosität besonders geeigneter, resorbierbarer Polymere, bevorzugt handelt es sich hierbei um resorbierbare Polyester, vorzugsweise um Milchsäurepolymere, insbesondere um Poly-D-, Poly-L- oder Poly-D,L-milchsäuren, gemessen in Chloroform bei 25°C, 0,1 % Polymerkonzentration, liegt im Bereich von 0,5 dl/g bis 8,0 dl/g, bevorzugt im Bereich von 0,8 dl/g bis 7,0 dl/g, insbesondere im Bereich von 1,5 dl/g bis 3,2 dl/g.

Weiterhin ist die inhärente Viskosität besonders geeigneter, resorbierbarer Polymere, bevorzugt handelt es sich hierbei um resorbierbare Polyester, günstigerweise um Milchsäurepolymere, insbesondere um Poly-D-, Poly-L- oder Poly-D,L-milchsäuren, gemessen in Hexafluor-2-propanol bei 30°C, 0,1 % Polymerkonzentration, im Bereich von 1,0 dl/g bis 2,6 dl/g, insbesondere im Bereich von 1,3 dl/g bis 2,3 dl/g.

Im Rahmen der vorliegenden Erfindung sind darüber hinaus Polymere, insbesondere thermoplastische Polymere, mit einer Glasübergangstemperatur größer 20°C, günstigerweise größer 25°C, bevorzugt größer 30°C, besonders bevorzugt größer 35°C, insbesondere größer 40°C, äußerst vorteilhaft. Im Rahmen einer ganz besonders bevorzugten Ausführungsform der vorliegenden Erfindung liegt die Glasübergangstemperatur des Polymers im Bereich von 35°C bis 55°C, insbesondere im Bereich von 40°C bis 50°C.

Weiterhin sind Polymere besonders geeignet, die eine Schmelztemperatur größer 50°C, günstigerweise von mindestens 60°C, bevorzugt von größer 150°C, besonders bevorzugt im Bereich von 160°C bis 210°C, insbesondere im Bereich von 175°C bis 195°C, aufweisen.

Dabei wird die Glastemperatur und die Schmelztemperatur des Polymers vorzugsweise mittels Dynamische Differenzkalorimetrie (Differential Scanning Calorimetry; kurz DSC) ermittelt. Ganz besonders bewährt hat sich in diesem Zusammenhang die folgende Vorgehensweise:
Durchführung der DSC-Messung unter Stickstoff auf einem Mettler-Toledo DSC 30S. Die Eichung erfolgt vorzugsweise mit Indium. Die Messungen werden vorzugsweise unter trockenem, Sauerstoff-freien Stickstoff (Strömungsgeschwindigkeit: vorzugsweise 40 ml/min) durchgeführt. Das Probengewicht wird vorzugsweise zwischen 15 mg und 20 mg gewählt. Die Proben werden zunächst von 0°C auf vorzugsweise eine Temperatur oberhalb der Schmelztemperatur des zu untersuchenden Polymers erwärmt, dann auf 0°C abgekühlt und ein zweites Mal von 0°C auf die genannte Temperatur mit einer Heizrate von 10°C/min erwärmt.

Ganz besonders bevorzugt als thermoplastische Polymere werden Polyamide sowie resorbierbare Polymere, vor allem resorbierbare Polyester, insbesondere Milchsäurepolymere.

Im Rahmen der vorliegenden Erfindung umfassen die kleinen Teilchen der Verbundteilchen mindestens Calciumcarbonat.

Die Form der Calciumcarbonat-Teilchen, insbesondere der gefällten Calciumcarbonat-Teilchen, unterliegt dabei keinen weiteren Beschränkungen und kann auf den konkreten Anwendungszweck abgestimmt werden. Bevorzugt werden jedoch skalenoedrische, rhomboedrische, nadelförmige, plättchenförmige oder kugelförmige (sphärische) Teilchen eingesetzt. Im Rahmen einer ganz besonders bevorzugten Ausführungsform der vorliegenden Erfindung werden kugelförmige Calciumcarbonat-, insbesondere kugelförmige, gefällte Calciumcarbonat-Teilchen, verwendet, da diese normalerweise ein isotropes Eigenschaftsprofil aufweisen. Dementsprechend zeichnen sich die resultierenden Verbundteilchen zweckmäßigerweise ebenfalls durch ein möglichst isotropes Eigenschaftsprofil aus.

Das Aspekt Ratio (Seitenverhältnis) der Calciumcarbonat-Teilchen, insbesondere der gefällten Calciumcarbonat-Teilchen, ist vorzugsweise kleiner 5, bevorzugt kleiner 4, besonders bevorzugt kleiner 3, günstigerweise kleiner 2, noch mehr bevorzugt kleiner 1,5, ganz besonders bevorzugt im Bereich von 1,0 bis 1,25, vorzugsweise kleiner 1,1, insbesondere kleiner 1,05.

Das Aspekt Ratio (Seitenverhältnis) der Calciumsalz-Teilchen bezeichnet in diesem Zusammenhang den Quotienten aus maximalem und minimalem Teilchendurchmesser. Es wird vorzugsweise mittels elektronenmikroskopischer Aufnahmen als Mittelwert (Zahlenmittel) ermittelt. In diesem Zusammenhang werden für kugelförmige Calciumsalz-Teilchen vorzugsweise nur Teilchen mit einer Teilchengröße im Bereich von 0,1 µm bis 30,0 µm berücksichtigt. Für rhomboedrische Calciumsalz-Teilchen werden vorzugsweise nur Teilchen mit einer Teilchengröße im Bereich von 0,1 µm bis 20,0 µm berücksichtigt. Für andere Calciumsalz-Teilchen werden vorzugsweise nur Teilchen mit einer Teilchengröße im Bereich von 0,1 µm bis 2,0 µm berücksichtigt.

Weiterhin weisen vorzugsweise mindestens 90 %, günstigerweise mindestens 95 % aller Teilchen, ein Aspekt Ratio (Seitenverhältnis) kleiner 5, bevorzugt kleiner 4, besonders bevorzugt kleiner 3, günstigerweise kleiner 2, noch mehr bevorzugt kleiner 1,5, ganz besonders bevorzugt im Bereich von 1,0 bis 1,25, vorzugsweise kleiner 1,1, insbesondere kleiner 1,05, auf.

Ganz besonders günstig sind weiterhin kugelförmige Calciumsalz-Teilchen, die vorzugsweise überwiegend einzelteilig vorliegen. Dabei werden kleinere Abweichungen von der perfekten Kugelform akzeptiert, solange die Eigenschaften der Teilchen nicht grundlegend verändert werden. So kann die Oberfläche der Teilchen gelegentliche Fehlstellen oder zusätzliche Ablagerungen aufweisen.

Im Rahmen einer besonders bevorzugten Variante der vorliegenden Erfindung sind die Calciumcarbonat-Teilchen, insbesondere die gefällten Calciumcarbonat-Teilchen, vorzugsweise kugelförmig und im Wesentlichen amorph. Der Begriff "amorph" bezeichnet an dieser Stelle solche Calciumsalz-Modifikationen, bei welchen die Atome zumindest teilweise keine geordneten Strukturen, sondern ein unregelmäßiges Muster ausbilden und daher nur über eine Nahordnung, nicht aber über eine Fernordnung verfügen. Hiervon zu unterscheiden sind kristalline Modifikationen des Calciumsalzes, wie z. B. Calcit, Vaterit und Aragonit, bei welchen die Atome sowohl eine Nah- als auch eine Fernordnung aufweisen.

Im Rahmen dieser bevorzugten Variante der vorliegenden Erfindung wird die Gegenwart von kristallinen Bestandteilen jedoch nicht kategorisch ausgeschlossen. Vorzugsweise ist der Anteil von kristallinen Calciumcarbonat jedoch kleiner 50 Gew.-%, besonders bevorzugt kleiner 30 Gew.-%, ganz besonders bevorzugt kleiner 15 Gew.-%, insbesondere kleiner 10 Gew.-%. Im Rahmen einer besonders bevorzugten Variante der vorliegenden Erfindung ist der Anteil von kristallinen Calciumcarbonat kleiner 8,0 Gew.-%, bevorzugt kleiner 6,0 Gew.-%, zweckmäßigerweise kleiner 4,0 Gew.-%, besonders bevorzugt kleiner 2,0 Gew.-%, ganz besonders bevorzugt kleiner 1,0 Gew.-%, insbesondere kleiner 0,5 Gew.-% , jeweils bezogen auf das Gesamtgewicht des Calciumsalzes.

Für die Ermittlung der amorphen und der kristallinen Anteile hat sich die Röntgenbeugung mit einem internen Standard, vorzugsweise Quarz, in Verbindung mit einer Rietveld-Verfeinerung ganz besonders bewährt.

Im Rahmen dieser bevorzugten Ausführungsform der vorliegenden Erfindung werden die Calciumcarbonat-Teilchen, bevorzugt die vorzugsweise amorphen Calciumcarbonat-Teilchen, günstigerweise durch mindestens eine Substanz, insbesondere mindestens eine grenzflächenaktive Substanz, stabilisiert, die vorzugsweise auf der Oberfläche der Calciumsalz-Teilchen, insbesondere auf der Oberfläche der vorzugsweise kugelförmigen Calciumcarbonat-Teilchen, angeordnet ist. "Grenzflächenaktive Substanzen" bezeichnen im Sinne der vorliegenden Erfindung zweckmäßigerweise organische Verbindungen, die sich aus ihrer Lösung an Grenzflächen (Wasser/Calciumsalz-Teilchen, bevorzugt Calciumcarbonat-Teilchen) stark anreichern und dadurch die Oberflächenspannung, vorzugsweise gemessen bei 25°C, herabsetzen. Für weitere Details wird auf die Fachliteratur, insbesondere auf Römpp-Lexikon Chemie / Hrsg. Jürgen Falbe; Manfred Regitz. Bearb. Von Eckard Amelingmeier; Stuttgart, New York; Thieme; Band 2: Cm-G; 10. Auflage (1997); Stichwort: "grenzflächenaktive Stoffe", verwiesen.

Vorzugsweise weist die Substanz, insbesondere die grenzflächenaktive Substanz, eine Molmasse größer 100 g/mol, bevorzugt größer 125 g/mol, insbesondere größer 150 g/mol, auf und genügt der Formel R-Xₙ.

Der Rest R steht dabei für einen mindestens 1, vorzugsweise mindestens 2, bevorzugt mindest 4, besonders bevorzugt mindestens 6, insbesondere mindestens 8, Kohlenstoffatome umfassenden Rest, vorzugsweise für einen aliphatischen oder cycloaliphatischen Rest, der ggf. weitere Reste X umfassen kann und der ggf. eine oder mehrere Etherverknüpfungen aufweisen kann.

Der Rest X steht für eine Gruppe, die mindestens ein Sauerstoffatom sowie mindestens ein Kohlenstoffatom, Schwefelatom, Phosphoratom und/oder Stickstoffatom, bevorzugt mindestens ein Phosphoratom und/oder mindestens ein Kohlenstoffatom, umfasst. Besonders bevorzugt werden die folgenden Gruppen:
Carbonsäuregruppen ∼COOH,
Carboxylatgruppen ∼COO⁻,
Sulfonsäuregruppen ~SO₃H,
Sulfonatgruppen ∼SO₃⁻,
Hydrogensulfatgruppen ∼OSO₃H,
Sulfatgruppen ∼OSO₃⁻,
Phosphonsäuregruppen ∼PO₃H₂,
Phosphonatgruppen ∼PO₃H⁻, ∼PO₃²⁻,
Aminogruppen ∼NR¹R² sowie
Ammoniumgruppen ∼N⁺R¹R²R³,
insbesondere Carbonsäuregruppen, Carboxylatgruppen, Phosphonsäuregruppen und Phosphonatgruppen.

Die Reste R¹, R² und R³ stehen in diesem Zusammenhang unabhängig von einander für Wasserstoff oder eine Alkylgruppe mit 1 bis 5 Kohlenstoffatomen. Einer der Reste R¹, R² und R³ kann auch ein Rest R sein.

Bevorzugte Gegenionen für die vorstehend genannten Anionen sind Metallkationen, insbesondere Alkalimetallkationen, bevorzugt Na⁺ und K⁺, sowie Ammoniumionen.

Bevorzugte Gegenionen für die vorstehend genannten Kationen sind Hydroxylionen, Hydrogencarbonationen, Carbonationen, Hydrogensulfationen, Sulfationen und Halogenidionen, insbesondere Chlorid- und Bromidionen.

n steht für eine vorzugsweise ganze Zahl im Bereich von 1 bis 20, bevorzugt im Bereich von 1 bis 10, insbesondere im Bereich von 1 bis 5.

Für die Zwecke der vorliegenden Erfindung besonders geeignete Substanzen umfassen Alkylcarbonsäuren, Alkylcarboxylate, Alkylsulfonsäuren, Alkylsulfonate, Alkylsulfate, Alkylethersulfate mit vorzugsweise 1 bis 4 Ethylenglykolethereinheiten, Fettalkoholethoxylate mit vorzugsweise 2 bis 20 Ethylenglykolethereinheiten, Alkylphenolethoxylate, ggf. substituierte Alkylphosphonsäuren, ggf. substituierte Alkylphosphonate, Sorbitanfettsäureester, Alkylpolyglucoside, N-Methylglucamide, Homo- und Copolymere der Acrylsäure sowie deren entsprechenden Salzformen und Blockcopolymere.

Eine erste Gruppe ganz besonders vorteilhafter Substanzen sind ggf. substituierte Alkylphosphonsäuren, insbesondere Amino-tri-(methylenphosphonsäure), 1-Hydroxyethylen-(1,1-diphosphonsäure), Ethylendiamin-tetra-(methylenphosphonsäure), Hexamethylendiamin-tetra-(methylenphosphonsäure), Diethylentriamin-penta-(methylenphosphonsäure), sowie ggf. substituierte Alkylphosphonate, insbesondere der vorstehend genannten Säuren. Diese Verbindungen sind als multifunktionelle Sequestriermittel für Metallionen und Steininhibitoren bekannt.

Weiterhin haben sich auch Homo- und Copolymere, bevorzugt Homopolymere, der Acrylsäure sowie deren entsprechenden Salzformen besonders bewährt, insbesondere solche, die ein Gewichtsmittel des Molekulargewichts im Bereich von 1.000 g/mol - 10.000 g/mol aufweisen.

Ferner ist die Verwendung von Blockcopolymeren, bevorzugt von doppelhydrophilen Blockcopolymeren, insbesondere von Polyethylenoxid oder Polypropylenoxid, besonders günstig.

Der Anteil der vorzugsweise grenzflächenaktiven Substanzen kann prinzipiell frei gewählt und für die jeweilige Anwendung gezielt eingestellt werden. Er liegt jedoch vorzugsweise im Bereich von 0,1 Gew.-% bis 5,0 Gew.-%, insbesondere im Bereich von 0,3 Gew.-% bis 1,0 Gew.-%, bezogen auf den Calciumsalz-Gehalt, insbesondere den CaCO₃-Gehalt, der Teilchen.

Die Herstellung der vorzugsweise kugelförmigen, vorzugsweise amorphen Calciumcarbonat-Teilchen kann auf an sich bekannte Weise z. B. durch Hydrolyse von Dialkylcarbonat oder von Alkylencarbonat in einer Calcium-Kationen umfassenden Lösung erfolgen.

Die Herstellung von nicht-stabilisierten, kugelförmigen Calciumcarbonat-Teilchen wird beispielsweise in der Patentanmeldung WO 2008/122358 im Detail beschrieben.

Die Hydrolyse des Dialkylcarbonats oder des Alkylencarbonats wird zweckmäßigerweise in Gegenwart eines Hydroxids durchgeführt.

Für die Zwecke der vorliegenden Beschreibung bevorzugte Substanzen, die Ca²⁺-lonen umfassen, sind Calciumhalogenide, bevorzugt CaCl₂, CaBr₂, insbesondere CaCl₂, sowie Calciumhydroxid. Im Rahmen einer ersten besonders bevorzugten Ausführungsform der vorliegenden Erfindung wird CaCl₂ eingesetzt. Im einer weiteren besonders bevorzugten Ausführungsform der vorliegenden Erfindung wird Ca(OH)₂ verwendet.

Im Rahmen einer ersten besonders bevorzugten Ausführungsform wird ein Dialkylcarbonat eingesetzt. Besonders geeignete Dialkylcarbonate umfassen 3 bis 20, bevorzugt 3 bis 9, Kohlenstoffatome, insbesondere Dimethylcarbonat, Diethylcarbonat, Di-n-propylcarbonat, Di-isopropylcarbonat, Di-n-butylcarbonat, Di-sec-butylcarbonat und Di-tert.-butylcarbonat, wobei Dimethylcarbonat in diesem Zusammenhang ganz besonders bevorzugt wird.

In einer weiteren besonders bevorzugten Ausführungsform wird ein Alkylencarbonat zur Reaktion gebracht. Besonders zweckmäßige Alkylencarbonate umfassen 3 bis 20, bevorzugt 3 bis 9, besonders bevorzugt 3 bis 6, Kohlenstoffatome und schließen insbesondere solche Verbindungen ein, die einen Ring aus 3 bis 8, bevorzugt 4 bis 6, insbesondere 5, Atomen, mit vorzugsweise 2 Sauerstoffatomen und ansonsten Kohlenstoffatomen, umfassen. Propylencarbonat (4-Methyl-1,3-dioxolan) hat sich in diesem Zusammenhang ganz besonders bewährt.
Als Hydroxid haben sich Alkalimetallhydroxide, insbesondere NaOH, und Calciumhydroxid, als besonders geeignet erwiesen. Im Rahmen einer ersten besonders bevorzugten Ausführungsform der vorliegenden Erfindung wird NaOH eingesetzt. Im Rahmen einer weiteren besonders bevorzugten Ausführungsform der vorliegenden Erfindung wird Ca(OH)₂ verwendet.

Weiterhin ist das molare Verhältnis von Ca²⁺, bevorzugt von Calciumchlorid, zu OH⁻, bevorzugt Alkalimetallhydroxid, in der Reaktionsmischung vorzugsweise größer 0,5 : 1 und besonders bevorzugt im Bereich von >0,5 : 1 bis 1 : 1, insbesondere im Bereich von 0,6 : 1 bis 0,9 : 1.

Das molare Verhältnis von Ca²⁺, bevorzugt von Calciumchlorid, zu Dialkylcarbonat und/oder Alkylencarbonat in der Reaktionsmischung liegt günstigerweise im Bereich von 0,9 : 1,5 bis 1,1 : 1, besonders bevorzugt im Bereich von 0,95 : 1 bis 1 : 0,95. Im Rahmen einer ganz besonders zweckmäßigen Variante der vorliegenden Erfindung werden das Dialkylcarbonat und/oder das Alkylencarbonat und das Ca²⁺, insbesondere das Calciumchlorid, äquimolar eingesetzt.

Im Rahmen einer ersten ganz besonders bevorzugten Variante wird nicht Ca(OH)₂ als OH⁻-Quelle verwendet. Die Komponenten für die Umsetzung setzt man dabei günstigerweise in folgenden Konzentrationen ein:
a) Ca²⁺: >10 mmol/l bis 50 mmol/l, bevorzugt 15 mmol/l bis 45 mmol/l, insbesondere 17 mmol/l bis 35 mmol/l;
b) Dialkylcarbonat und/oder
   Alkylencarbonat: >10 mmol/l bis 50 mmol/l, bevorzugt 15 mmol/l bis 45 mmol/l, insbesondere 17 mmol/l bis 35 mmol/l;
c) OH⁻: 20 mmol/l bis 100 mmol/l, bevorzugt 20 mmol/l bis 50 mmol/l, besonders bevorzugt 25 mmol/l bis 45 mmol/l, insbesondere 28 mmol/l bis 35 mmol/l.

Die jeweiligen Konzentrationsangaben beziehen sich dabei auf die Konzentrationen der genannten Komponenten in der Reaktionsmischung.

Im Rahmen einer weiteren ganz besonders bevorzugten Variante wird Ca(OH)₂, bevorzugt Kalkmilch, insbesondere gesättigte Kalkmilch, als OH⁻-Quelle verwendet. Die Komponenten für die Umsetzung setzt man dabei günstigerweise in folgenden Konzentrationen ein:
a) Ca(OH)₂: >5 mmol/l bis 25 mmol/l, bevorzugt 7,5 mmol/l bis 22,5 mmol/l, insbesondere 8,5 mmol/l bis 15,5 mmol/l;
b) Dialkylcarbonat und/oder
Alkylencarbonat: >5 mmol/l bis 25 mmol/l, bevorzugt 7,5 mmol/l bis 22,5 mmol/l, insbesondere 8,5 mmol/l bis 15,5 mmol/l.

Die jeweiligen Konzentrationsangaben beziehen sich dabei auf die Konzentrationen der genannten Komponenten in der Reaktionsmischung. Die Reaktion der Komponenten wird vorzugsweise bei einer Temperatur im Bereich von 15°C bis 30°C durchgeführt.

Die konkrete Größe der Calciumcarbonat-Teilchen kann auf an sich bekannte Weise über die Übersättigung gesteuert werden.

Die Calciumcarbonat-Teilchen fallen unter den oben genannten Bedingungen aus der Reaktionsmischung aus.

Die Stabilisierung der vorzugsweise amorphen Calciumcarbonat-Teilchen erfolgt zweckmäßigerweise durch Zugabe der vorzugsweise grenzflächenaktiven Substanz zu der Reaktionsmischung.

Diese Zugabe der Substanz sollte dabei erst nach Beginn der Reaktion zur Bildung der Calciumcarbonat-Teilchen, d. h. erst nach Zugabe der Edukte, vorzugsweise frühestens 1 Minute, bevorzugt frühestens 2 Minuten, zweckmäßigerweise frühestens 3 Minuten, besonders bevorzugt frühestens 4 Minute, insbesondere frühestens 5 Minuten, nach Mischen der Edukte erfolgen. Weiterhin sollte der Zeitpunkt der Zugabe so gewählt werden, dass die vorzugsweise grenzflächenaktive Substanz kurz vor dem Ende der Fällung und möglichst kurz vor dem Beginn der Umwandlung des vorzugsweise amorphen Calciumcarbonats in eine kristalline Modifikationen zuzugeben wird, da sich auf diese Weise die Ausbeute und die Reinheit der "*stabilisierten, kugelförmigen, amorphen Calciumsalz-Teilchen*" maximieren lässt. Erfolgt die Zugabe der vorzugsweise grenzflächenaktiven Substanz früher, so wird in der Regel ein bimodales Produkt erhalten, dass neben den gewünschten, stabilisierten, kugelförmigen, amorphen Calciumsalz-Teilchen ultrafeine, amorphe Calciumsalz-Teilchen als Nebenprodukt umfasst. Erfolgt die Zugabe der vorzugsweise grenzflächenaktiven Substanz später, so setzt bereits die Umwandlung der gewünschten "*stabilisierten Calciumsalz*-*Teilchen*" in kristalline Modifikationen ein.

Aus diesem Grund wird die vorzugsweise grenzflächenaktive Substanz vorzugsweise bei einem pH-Wert kleiner gleich 11,5, bevorzugt kleiner gleich 11,3, insbesondere kleiner gleich 11,0, zugegeben. Besonders günstig ist eine Zugabe bei einem pH-Wert im Bereich von 11,5 bis 10,0, bevorzugt im Bereich von 11,3 bis 10,5, insbesondere im Bereich von 11,0 bis 10,8, jeweils gemessen bei der Reaktionstemperatur, vorzugsweise bei 25°C.

Die resultierenden, stabilisierten, vorzugsweise kugelförmigen, amorphen Calciumcarbonat-Teilchen können auf an sich bekannte Weise, z. B. durch Zentrifugation, entwässert und getrocknet werden. Ein Waschen mit Aceton und/oder eine Trocknung im Vakuumtrockenschrank ist nicht mehr unbedingt erforderlich.

Durch Trocknung der sind aus den "*stabilisierten* Calciumcarbonat-Teilchen" "*Calciumcarbonat-Teilchen mit geringem* Strukturwassergehalt" erhältlich.

Für die Zwecke der vorliegenden Erfindung werden die erhaltenen Calciumcarbonat-Teilchen vorzugsweise derart getrocknet, dass sie den gewünschten Restwassergehalt aufweisen. Hierfür hat sich eine Vorgehensweise besonders bewährt, bei welcher man die Calciumcarbonat-Teilchen vorzugsweise zunächst bei einer Temperatur bis zu 150°C vortrocknet und anschließend die Calciumsalz-Teilchen vorzugsweise bei einer Temperatur im Bereich von größer 150°C bis 250°C, bevorzugt im Bereich von 170°C bis 230°C, besonders bevorzugt im Bereich von 180°C bis 220°C, insbesondere im Bereich von 190°C bis 210°C, trocknet. Die Trocknung erfolgt vorzugsweise im Umlufttrockenschrank. Dabei werden die Calciumcarbonat-Teilchen zweckmäßigerweise mindestens 3 h, besonders bevorzugt mindestens 6 h, insbesondere mindestens 20 h getrocknet. Im Rahmen einer weiteren besonders bevorzugten Variante der vorliegenden Erfindung sind die gefällten Calciumcarbonat-Teilchen im Wesentlichen kristallin, insbesondere im Wesentlichen calcitisch. Im Rahmen dieser bevorzugten Variante der vorliegenden Erfindung wird die Gegenwart von anderen, insbesondere von amorphen Bestandteilen jedoch nicht kategorisch ausgeschlossen. Vorzugsweise ist der Anteil von anderen nicht-kristallinen Calciumsalzmodifikationen, insbesondere von anderen nicht-kristallinen Calciumcarbonatmodifikationen, jedoch kleiner 50 Gew.-%, besonders bevorzugt kleiner 30 Gew.-%, ganz besonders bevorzugt kleiner 15 Gew.-%, insbesondere kleiner 10 Gew.-%. Weiterhin ist der Anteil von nicht-calcitischen Calciumcarbonatmodifikationen vorzugsweise kleiner 50 Gew.-%, besonders bevorzugt kleiner 30 Gew.-%, ganz besonders bevorzugt kleiner 15 Gew.-%, insbesondere kleiner 10 Gew.-%.

Der mittlere Durchmesser der gefällten Calciumcarbonat-Teilchen liegt im Bereich 0,01 µm bis 1,0 mm und vorzugsweise im Bereich von 0,05 µm bis 30,0 µm, insbesondere im Bereich von 0,1 µm bis 15,0 µm.

Für amorphe Calciumcarbonat-Teilchen ist der mittlere Durchmesser der Calciumsalz-Teilchen günstigerweise im Bereich von 0,05 µm bis 2,0 µm, vorzugsweise kleiner 1,75 µm, besonders bevorzugt kleiner 1,5 µm, insbesondere kleiner 1,2 µm. Weiterhin ist der mittlere Teilchendurchmesser in diesem Fall günstigerweise größer 0,1 µm, vorzugsweise größer 0,2 µm, insbesondere größer 0,3 µm.

Für skalenoedrische Calciumcarbonat-Teilchen ist der mittlere Durchmesser der Calciumsalz-Teilchen günstigerweise im Bereich von 0,05 µm bis 2,0 µm, vorzugsweise kleiner 1,75 µm, besonders bevorzugt kleiner 1,5 µm, insbesondere kleiner 1,2 µm. Weiterhin ist der mittlere Teilchendurchmesser in diesem Fall günstigerweise größer 0,1 µm, vorzugsweise größer 0,2 µm, insbesondere größer 0,3 µm.

Weiterhin haben sich auch skalenoedrische Calciumcarbonat-Teilchen besonders bewährt, die einen mittleren Durchmesser günstigerweise im Bereich von 1,0 µm bis 5,0 µm, vorzugsweise kleiner 4,5 µm, besonders bevorzugt kleiner 4,0 µm, insbesondere kleiner 3,5 µm, aufweisen. Weiterhin ist der mittlere Teilchendurchmesser in diesem Fall günstigerweise größer 1,5 µm, vorzugsweise größer 2,0 µm, insbesondere größer 3,0 µm.

Für rhomboedrische Calciumcarbonat-Teilchen ist der mittlere Durchmesser der Calciumsalz-Teilchen günstigerweise im Bereich von 0,05 µm bis 2,0 µm, vorzugsweise kleiner 1,75 µm, besonders bevorzugt kleiner 1,5 µm, insbesondere kleiner 1,2 µm. Weiterhin ist der mittlere Teilchendurchmesser in diesem Fall günstigerweise größer 0,1 µm, vorzugsweise größer 0,2 µm, insbesondere größer 0,3 µm.

Weiterhin haben sich auch rhomboedrische Calciumcarbonat-Teilchen besonders bewährt, die einen mittleren Durchmesser günstigerweise im Bereich von 1,0 µm bis 20,0 µm, vorzugsweise kleiner 18,0 µm, besonders bevorzugt kleiner 16,0 µm, insbesondere kleiner 14,0 µm, aufweisen.

Weiterhin ist der mittlere Teilchendurchmesser in diesem Fall günstigerweise größer 2,5 µm, vorzugsweise größer 4,0 µm, insbesondere größer 6,0 µm.

Für nadelförmige Calciumcarbonat-Teilchen ist der mittlere Durchmesser der Calciumsalz-Teilchen günstigerweise im Bereich von 0,05 µm bis 2,0 µm, vorzugsweise kleiner 1,5 µm, besonders bevorzugt kleiner 1,0 µm, insbesondere kleiner 0,75 µm. Weiterhin ist der mittlere Teilchendurchmesser in diesem Fall günstigerweise größer 0,1 µm, vorzugsweise größer 0,2 µm, insbesondere größer 0,3 µm.

Für plättchenförmige Calciumcarbonat-Teilchen ist der mittlere Durchmesser der Calciumsalz-Teilchen günstigerweise im Bereich von 0,05 µm bis 2,0 µm, vorzugsweise kleiner 1,75 µm, besonders bevorzugt kleiner 1,5 µm, insbesondere kleiner 1,2 µm. Weiterhin ist der mittlere Teilchendurchmesser in diesem Fall günstigerweise größer 0,1 µm, vorzugsweise größer 0,2 µm, insbesondere größer 0,3 µm.

Für sphärolithische (kugelförmige) Calciumcarbonat-Teilchen hat sich weiterhin ein mittlerer Durchmesser günstigerweise im Bereich von 1,0 µm bis 30,0 µm, bevorzugt kleiner 20,0 µm, vorzugsweise kleiner 18,0 µm, besonders bevorzugt kleiner 16,0 µm, insbesondere kleiner 14,0 µm, besonders bewährt. Weiterhin ist der mittlere Teilchendurchmesser in diesem Fall günstigerweise größer 2,5 µm, vorzugsweise größer 4,0 µm, insbesondere größer 6,0 µm.

Die vorstehend genannten mittleren Teilchengrößen der Calciumcarbonat-Teilchen werden im Rahmen der vorliegenden Erfindung zweckmäßigerweise durch Auswertung von Rasterelektronenmikroskop-Aufnahmen (REM-Aufnahmen) ermittelt, wobei vorzugsweise nur Teilchen mit einer Größe von mindestens 0,01 µm berücksichtigt werden und ein Zahlenmittel über vorzugsweise mindestens 20, besonders bevorzugt mindestens 40 Teilchen gebildet wird. Weiterhin haben sich auch Sedimentationsanalyseverfahren, vor allem für nadelförmige Calciumsalz-Teilchen besonders bewährt, wobei in diesem Zusammenhang die Verwendung eines Sedigraphs 5100 (Micromeritics GmbH) besonders vorteilhaft ist. Bei nicht-kugelförmigen Calciumsalz-Teilchen wird vorzugsweise auf die kugeläquivalente Teilchengröße abgestellt.

Die Größenverteilung der Calciumcarbonat-Teilchen ist vergleichsweise eng und vorzugsweise derart, dass mindestens 90,0 Gew.-% aller Calciumcarbonat-Teilchen einen Teilchendurchmesser im Bereich von mittlerer Teilchendurchmesser -50 %, bevorzugt im Bereich von mittlerer Teilchendurchmesser -40 %, insbesondere im Bereich von mittlerer Teilchendurchmesser -30 %, bis mittlerer Teilchendurchmesser +70 %, bevorzugt mittlerer Teilchendurchmesser +60 %, insbesondere mittlerer Teilchendurchmesser +50 %, aufweisen. Dabei wird die Größenverteilung vorzugsweise mittels Rastertunnelmikroskopie ermittelt.

Der Formfaktor der Calciumcarbonat-Teilchen vorliegend definiert als der Quotient aus minimalem Teilchendurchmesser und maximalem Teilchendurchmesser, ist zweckmäßigerweise für mindestens 90 %, günstigerweise für mindestens 95 % aller Teilchen größer 0,90, besonders bevorzugt größer 0,95. In diesem Zusammenhang werden für kugelförmige Calciumcarbonat-Teilchen vorzugsweise nur Teilchen mit einer Teilchengröße im Bereich von 0,1 µm bis 30,0 µm berücksichtigt. Für rhomboedrische Calciumcarbonat-Teilchen werden vorzugsweise nur Teilchen mit einer Teilchengröße im Bereich von 0,1 µm bis 20,0 µm berücksichtigt. Für andere Calciumcarbonat-Teilchen werden vorzugsweise nur Teilchen mit einer Teilchengröße im Bereich von 0,1 µm bis 2,0 µm berücksichtigt.

Die Calciumcarbonat-Teilchen zeichnen sich günstigerweise weiterhin durch einen vergleichsweise geringen Wassergehalt aus. Sie weisen, bezogen auf ihr Gesamtgewicht, einen Wassergehalt (Restfeuchte bei 200°C) von höchstens 5,0 Gew.-%, vorzugsweise von höchstens 2,5 Gew.-%, bevorzugt von höchstens 1,0 Gew.-%, besonders bevorzugt von höchstens 0,5 Gew.-%, noch mehr bevorzugt kleiner 0,4 Gew.-%, zweckmäßigerweise kleiner 0,3 Gew.-%, günstigerweise kleiner 0,2 Gew.-%, insbesondere von höchstens 0,1 Gew.-%, auf.

Im Rahmen der vorliegenden Erfindung wird der Wassergehalt der Calciumcarbonat-Teilchen vorzugsweise mittels Thermogravimetrie ermittelt, wobei die Messung vorzugsweise unter Stickstoff (Stickstoff-Durchflußmenge bevorzugt 20 ml/min) und zweckmäßigerweise über den Temperaturbereich von 40°C oder niedriger bis 250°C oder höher durchgeführt wird. Weiterhin erfolgt die Messung vorzugsweise bei einer Heizrate von 10°C/min.

Werden im Rahmen der vorliegenden Erfindung amorphe Calciumcarbonat-Teilchen eingesetzt, so weisen diese günstigerweise einen geringen Gehalt an Strukturwasser auf. Dieser ist vorzugsweise kleiner 5 mol, bevorzugt kleiner 3 mol, besonders bevorzugt kleiner 1 mol, insbesondere kleiner 0,5 mol Strukturwasser pro mol Calciumcarbonat. In einer ganz besonders bevorzugten Ausführungsform der vorliegenden Erfindung umfassen die amorphen Calciumcarbonat-Teilchen kein Strukturwasser.

Die spezifische Oberfläche der Calciumcarbonat-Teilchen liegt vorzugsweise im Bereich von 0,1 m²/g bis 100 m²/g, besonders bevorzugt im Bereich von 0,1 m²/g bis 20,0 m²/g, insbesondere im Bereich von 4,0 m²/g bis 12,0 m²/g. Für rhomboedrische Calciumsalz-Teilchen, insbesondere für rhomboedrische Calciumcarbonat-Teilchen ist die spezifische Oberfläche im Rahmen einer besonders bevorzugten Variante der vorliegenden Erfindung kleiner 1,0 m²/g, bevorzugt kleiner 0,75 m²/g, insbesondere kleiner 0,5 m²/g, wobei der mittlere Durchmesser der rhomboedrischen Calciumsalz-Teilchen, insbesondere der rhomboedrischen Calciumcarbonat-Teilchen, günstigerweise größer 2,5 µm, vorzugsweise größer 4,0 µm, insbesondere größer 6,0 µm, ist.

Für kugelförmige Calciumcarbonat-Teilchen, welche vorzugsweise einen mittleren Durchmesser größer 2,5 µm, vorzugsweise größer 4,0 µm, insbesondere größer 6,0 µm, aufweisen, ist die spezifische Oberfläche im Rahmen einer besonders bevorzugten Variante der vorliegenden Erfindung kleiner 3,0 m²/g, bevorzugt kleiner 2,0 m²/g, insbesondere kleiner 1,5 m²/g. Weiterhin ist die spezifische Oberfläche in diesem Fall günstigerweise größer 0,25 m²/g, vorzugsweise größer 0,5 m²/g, insbesondere größer 0,75 m²/g.

Ganz besonders bevorzugt werden in diesem Zusammenhang Calciumcarbonat-Teilchen, deren spezifische Oberfläche während einer Trocknung relativ konstant bleibt und sich vorzugsweise um maximal 200 %, bevorzugt um maximal 150 %, insbesondere um maximal 100 %, jeweils bezogen auf den Ausgangswert, ändert.

Die Basizität der Calciumcarbonat-Teilchen ist vergleichsweise gering. Ihr pH-Wert, gemessen nach EN ISO 787-9, ist vorzugsweise kleiner 11,5, bevorzugt kleiner 11,0, insbesondere kleiner 10,5.

Bei den Verbundteilchen nach Anspruch 1 umfassen die kleinen Teilchen erfindungsgemäß gefällte Calciumcarbonat-Teilchen mit einer mittleren Teilchengröße im Bereich von 0,01 µm bis 1,0 mm.

Im Rahmen der vorliegenden Erfindung sind die mikrostrukturierten Verbundteilchen durch ein Verfahren erhältlich, bei welchem man große Teilchen mit kleinen Teilchen verbindet, wobei
- die großen Teilchen einen mittleren Teilchendurchmesser im Bereich von 0,1 µm bis 10 mm, bevorzugt im Bereich von 0,5 µm bis 2,0 mm, insbesondere im Bereich von 1,0 µm bis 500,0 µm, aufweisen,
- der mittlere Teilchendurchmesser der kleinen Teilchen höchstens 1/10 des mittleren Teilchendurchmessers der großen Teilchen ist und vorzugsweise im Bereich von 0,01 µm bis 1,0 mm, bevorzugt im Bereich von 0,02 µm bis 200 µm, zweckmäßigerweise im Bereich von 0,05 µm bis 30,0 µm, insbesondere im Bereich von 0,1 µm bis 15,0 µm, liegt.

Dabei werden die kleinen Teilchen auf der Oberfläche der großen Teilchen angeordnet und/oder inhomogen innerhalb der großen Teilchen verteilt.

Eine "inhomogene" Verteilung der kleinen Teilchen innerhalb der großen Teilchen bedeutet hier eine nicht homogene (gleichmäßige) Verteilung der kleinen Teilchen innerhalb der großen Teilchen. Vorzugsweise gibt es innerhalb der Verbundteilchen mindestens einen ersten Bereich, der mindestens zwei, vorzugsweise mindestens drei, bevorzugt mindestens vier, insbesondere mindestens fünf kleine Teilchen umfasst, und mindestens einen anderen Bereich innerhalb der Verbundteilchen, der zwar das gleiche Volumen und die gleiche Form wie der erste Bereich aufweist, aber eine andere Anzahl von kleinen Teilchen umfasst.

Weiterhin ist das Gewichtsverhältnis Polymer zu gefälltes Calciumcarbonat im Teilcheninneren größer als das Gewichtsverhältnis Polymer zu gefälltes Calciumcarbonat im Außenbereich der Teilchen. Zweckmäßigerweise ist das Gewichtsverhältnis Polymer zu gefälltes Calciumcarbonat im Teilcheninneren größer 50:50, bevorzugt größer 60:40, günstigerweise größer 70:30, besonders bevorzugt größer 80:20, noch mehr bevorzugt größer 90:10, ganz besonders bevorzugt größer 95:5, insbesondere größer 99:1. Weiterhin ist das Gewichtsverhältnis gefälltes Calciumcarbonat zu Polymer im Außenbereich der Teilchen, bevorzugt im Vorzugsaußenbereich der Teilchen, größer 50:50, bevorzugt größer 60:40, günstigerweise größer 70:30, besonders bevorzugt größer 80:20, noch mehr bevorzugt größer 90:10, ganz besonders bevorzugt größer 95:5, insbesondere größer 99:1.

Das Teilcheninnere bezeichnet in diesem Zusammenhang den Bereich, der vom Teilchenmittelpunkt maximal einen Abstand r/2 aufweist, wobei der Teilchenmittelpunkt vorzugsweise dem Schwerpunkt des Teilchen entspricht und wobei r dem Radius des kompakten kugelförmigen Teilchens entspricht, das aus den gleichen Komponenten in den gleichen Mengenverhältnissen und mit den gleichen Dichten besteht.

Der Außenbereich der Teilchen bezeichnet den Bereich, der vom Teilchenmittelpunkt einen Abstand größer r/2 aufweist. Der Vorzugsaußenbereich der Teilchen bezeichnet den Bereich, der vom Teilchenmittelpunkt einen Abstand größer 0,793 r aufweist. Dabei sind der Teilchenmittelpunkt und r jeweils wie zuvor definiert.

Für die Zwecke der vorliegenden Erfindung besonders bevorzugte Verbundteilchen sind kugelförmig. Der mittlere Durchmesser der Verbundteilchen liegt vorzugsweise im Bereich von 0,1 µm bis 12 mm, insbesondere im Bereich von 0,5 µm bis 2,4 mm.

Im Rahmen einer besonders bevorzugten Ausführungsform der vorliegenden Erfindung umfassen die erfindungsgemäßen Verbundteilchen einen Kern und eine Hülle, wobei der Kern vorzugsweise mindestens ein Polymer, insbesondere mindestens ein thermoplastisches Polymer, umfasst und vorzugsweise einen mittleren Durchmesser im Bereich von 0,1 µm bis 10 mm, insbesondere im Bereich von 0,5 µm bis 2 mm, aufweist. Die Hülle umfasst mindestens ein gefälltes Calciumcarbonat, und weist vorzugsweise eine mittlere Dicke von höchstens 20 %, bezogen auf den Kerndurchmesser, auf. Die mittleren Durchmesser der Verbundteilchen, der großen Teilchen und der kleinen Teilchen sowie die vorstehend genannten Längengrößen werden erfindungsgemäß zweckmäßigerweise anhand mikroskopischer Aufnahmen, ggf. anhand elektronenmikroskopischer Aufnahmen, ermittelt. Für die Ermittlung der mittleren Durchmesser der großen Teilchen und der kleinen Teilchen sind auch Sedimentationsanalysen besonders vorteilhaft, wobei hier die Verwendung eines Sedigraphs 5100 (Micromeritics GmbH) besonders günstig ist. Für die Verbundteilchen haben sich auch Partikelgrößenanalysen mit Laserbeugung besonders bewährt, wobei in diesem Zusammenhang die Verwendung eines Laserbeugungssensors HELOS/BR der Firma Sympatec GmbH besonders vorteilhaft ist. Dieser umfasst vorzugsweise einen RODOS Trockendispergierer. Bei nicht-kugelförmigen Teilchen wird vorzugsweise auf die kugeläquivalente Teilchengröße abgestellt.

Im Übrigen beziehen sich diese Angaben sowie alle anderen Angaben in der vorliegenden Beschreibung, sofern nichts anderes angegeben wird, auf eine Temperatur von 25°C.

Die erfindungsgemäßen Verbundteilchen sind vergleichsweise kompakt. Vorzugsweise ist der Anteil von Teilbereichen im Inneren der Teilchen, die eine Dichte kleiner 0,5 g/cm³, insbesondere kleiner 0,25 g/cm³, aufweisen, kleiner 10,0 %, bevorzugt kleiner 5,0 %, insbesondere kleiner 1,0 %, jeweils bezogen auf das Gesamtvolumen der Verbundteilchen.

Der Gewichtsanteil des gefällten Calciumcarbonats, bezogen auf das Gesamtgewicht der Verbundteilchen, beträgt vorzugsweise mindestens 0,1 Gew.-%, bevorzugt mindestens 1,0 Gew.-%, besonders bevorzugt mindestens 5,0 Gew.-%, und liegt zweckmäßigerweise im Bereich von 5,0 Gew.-% bis 50,0 Gew.-%, günstigerweise im Bereich von 10,0 Gew.-% bis 30,0 Gew.-%, insbesondere im Bereich von 15,0 Gew.-% bis 25,0 Gew.-%.

Der Gewichtsanteil des Polymer, bevorzugt des thermoplastischen Polymers, bezogen auf das Gesamtgewicht der Verbundteilchen, beträgt vorzugsweise mindestens 0,1 Gew.-%, bevorzugt mindestens 1,0 Gew.-%, besonders bevorzugt mindestens 5,0 Gew.-%, und liegt zweckmäßigerweise im Bereich von 5,0 Gew.-% bis 95,0 Gew.-%, günstigerweise im Bereich von 70,0 Gew.-% bis 90,0 Gew.-%, insbesondere im Bereich von 75,0 Gew.-% bis 85,0 Gew.-%.

Die erfindungsgemäßen Verbundteilchen zeichnen sich unter anderem durch eine sehr gute Verbindung der kleinen Teilchen mit den großen Teilchen aus. Die feste Verbindung der kleinen Teilchen mit den großen Teilchen lässt sich vorzugsweise durch mechanische Beanspruchung der Verbundteilchen, insbesondere durch Ausschütteln der Verbundteilchen mit Wasser bei 25°C, vorzugsweise gemäß der in Organikum, 17. Auflage, VEB Deutscher Verlag der Wissenschaften, Berlin, 1988, Abschnitt 2.5.2.1 "Ausschütteln von Lösungen bzw. Suspensionen", Seite 56-57 beschriebenen Vorgehensweise, verifizieren. Die Schüttelzeit beträgt vorzugsweise mindestens eine Minute, bevorzugt mindestens 5 Minuten, insbesondere 10 Minuten, und führt vorzugsweise nicht zu einer wesentlichen Veränderung der Form, der Größe und/oder der Zusammensetzung der Verbundteilchen. Besonders bevorzugt sind nach dem Schütteltest mindestens 60 Gew.-%, vorzugsweise mindestens 70 Gew.-%, bevorzugt mindestens 80 Gew.-%, besonders bevorzugt mindestens 90 Gew.-%, günstigerweise mindestens 95 Gew.-%, insbesondere mindestens 99 Gew.-% der Verbundteilchen, hinsichtlich ihrer Zusammensetzung, ihrer Größe und vorzugsweise ihrer Form nicht verändert.

Die Herstellung der erfindungsgemäßen Verbundteilchen kann auf an sich bekannte Weise, beispielsweise durch ein einstufiges Verfahren, insbesondere durch Auffällen oder Beschichten, vorzugsweise durch Beschichten mit Mahlgut, erfolgen.

Besonders bewährt hat sich jedoch eine Vorgehensweise, bei welcher man Polymerpartikel und Calciumcarbonatpartikel miteinander in Kontakt bringt und durch Einwirkung mechanischer Kräfte miteinander verbindet. Zweckmäßigerweise erfolgt dies in einem geeigneten Mischer oder in einer Mühle, insbesondere in einer Prallmühle, Stiftmühle oder in einer Ultrarotormühle. Die Rotorgeschwindigkeit ist dabei vorzugsweise größer 1 m/s, bevorzugt, größer 10 m/s, besonders bevorzugt größer 25 m/s, insbesondere im Bereich von 50 m/s bis 100 m/s.

Gemäß einer besonders bevorzugten Ausführungsform der vorliegenden Erfindung führt diese Vorgehensweise dazu, dass die Calciumcarbonat-Partikel in das Innere der Polymerpartikel eindringen und von dem Polymer möglichst vollständig bedeckt werden, so dass sie von außen nicht erkennbar sind. Derartige Teilchen können wie das Polymer ohne Calciumsalz-Partikel verarbeitet und verwendet werden, weisen aber die verbesserten Eigenschaften der erfindungsgemäßen Verbundteilchen auf.

Gemäß einer weiteren besonders bevorzugten Ausführungsform der vorliegenden Erfindung führt diese Vorgehensweise nicht dazu, dass die Calciumcarbonat-Partikel in das Innere der Polymerpartikel eindringen, sondern die Calciumsalz-Partikel werden vielmehr an der Oberfläche der Polymerpartikel fixiert und sind von außen leicht erkennbar.

Im Rahmen einer ersten besonders bevorzugten Variante Erfindung erfolgt die Herstellung der Verbundteilchen in Anlehnung an die in der Patentanmeldung JP62083029 A beschriebenen Vorgehensweise. Dabei werden große Teilchen (sogenannte Mutterpartikel) mit kleinen Teilchen (sogenannte Babypartikel) auf der Oberfläche beschichtet. Zu diesem Zweck wird vorzugsweise eine Oberflächenmodifizierungsvorrichtung ("*Hybridizer*") eingesetzt, die einen Hochgeschwindigkeitsrotor, einen Stator und ein kugelförmiges Gefäß, vorzugsweise umfassend innenliegende Messer, umfasst. Der Einsatz von NARA Hybridization Systemen, die vorzugsweise einen Rotor-Aussendurchmeser von 118 mm aufweisen, insbesondere von einem Hybridization System mit der Bezeichnung NHS-0 oder NHS-1 der Firma NARA Machinery Co., Ltd., hat sich in diesem Zusammenhang besonders bewährt.

Die Mutterpartikel und die Babypartikel werden gemischt, vorzugsweise feinstverteilt und in den Hybridizer eingebracht. Dort wird die Mischung vorzugsweise weiter feinstverteilt und vorzugsweise wiederholt mechanischen Kräften ausgesetzt, insbesondere Stoßkräften, Kompressionskräften, Reibungskräften und Scherkräften sowie den gegenseitigen Wechselwirkungen der Teilchen, um die Babyteilchen in den Mutterteilchen einheitlich einzubetten. Bevorzugte Rotorgeschwindigkeiten liegen im Bereich von 50 m/s bis 100 m/s, bezogen auf die Umfangsgeschwindigkeit.

Für weitere Details zu diesem Verfahren, insbesondere bezüglich der besonders zweckmäßigen Ausführungsformen, wird auf die JP62083029 A verwiesen.

Im Rahmen einer weiteren besonders bevorzugten Variante erfolgt die Herstellung der Verbundteilchen in Anlehnung an die in der Patentanmeldung DE 42 44 254 A1 beschriebenen Vorgehensweise. Dementsprechend ist ein Verfahren zur Herstellung von Verbundteilchen durch Befestigen einer Substanz auf der Oberfläche eines thermoplastischen Materials besonders günstig, wenn das thermoplastische Material einen durchschnittlichen Teilchendurchmesser von 100 µm bis 10 mm hat und die Substanz einen geringeren Teilchendurchmesser und eine bessere Hitzebeständigkeit hat als das thermoplastische Material, insbesondere wenn das Verfahren die Schritte umfasst:
o zuerst Erhitzen der Substanz, die den geringeren Teilchendurchmesser und die bessere Hitzebeständigkeit hat als das thermoplastische Material, auf eine Temperatur, die vorzugsweise nicht kleiner ist als der Erweichungspunkt des thermoplastischen Materials, unter Rühren in einer Vorrichtung, die vorzugsweise ein Rührwerk und eine Heizeinrichtung hat;
o Zugeben des thermoplastischen Materials in die Vorrichtung; und
o Befestigen der Substanz mit der besseren Hitzebeständigkeit auf der Oberfläche des thermoplastischen Materials.

Für weitere Details zu diesem Verfahren, insbesondere bezüglich der besonders zweckmäßigen Ausführungsformen, wird auf die DE 42 44 254 A1 verwiesen.

Im Rahmen noch einer weiteren besonders bevorzugten Variante erfolgt die Herstellung der Verbundteilchen in Anlehnung an die in der Patentanmeldung EP 0 922 488 A1 und/oder in dem Patent US 6,403,219 B1 beschriebenen Vorgehensweise. Dementsprechend ist ein Verfahren zur Herstellung von Verbundteilchen durch Befestigung oder Ankleben von feinen Partikeln auf der Oberfläche eines Feststoffpartikels, das als ein Kern fungiert, durch Anwendung eines Aufpralls und anschließendes Wachstum von einem oder mehreren Kristallen auf der Kernoberfläche besonders vorteilhaft. Für weitere Details zu diesem Verfahren, insbesondere bezüglich der besonders zweckmäßigen Ausführungsformen, wird auf die Patentanmeldung EP 0 922 488 A1 und/oder das Patent US 6,403,219 B1 verwiesen.

Im Rahmen einer weiteren besonders bevorzugten Ausführungsform werden die Verbundteilchen einer Fixierung in Anlehnung an die in der Patentanmeldung EP 0 523 372 A1 beschriebenen Vorgehensweise unterzogen. Diese ist insbesondere für Verbundteilchen zweckmäßig, die in Anlehnung an das in der Patentanmeldung JP62083029 A beschriebene Verfahren erhalten wurden. Die Fixierung der Verbundteilchen erfolgt dabei vorzugsweise durch thermisches Plasmaspritzen, wobei vorzugsweise eine Unterdruckplasmasprühvorrichtung ("reduced pressure plasma spraying device") eingesetzt wird, die vorzugsweise eine Leistung von mindestens 30 kW aufweist, insbesondere das in EP 0 523 372 A1 beschriebene Gerät.

Für weitere Details zu diesem Verfahren, insbesondere bezüglich der besonders zweckmäßigen Ausführungsformen, wird auf die Patentanmeldung EP 0 523 372 A1 verwiesen.

Die erfindungsgemäßen Verbundteilchen zeichnen sich durch ein hervorragendes Eigenschaftsprofil aus. Neben hervorragenden mechanischen Eigenschaften weisen sie eine sehr gute Dispergierbarkeit, eine überragende Mahlbarkeit, eine äußerst geringe Staubneigung sowie eine vergleichsweise hohe Isotropie auf.

Weiterhin ermöglicht die Anwesenheit des Calciumcarbonates in den Verbundteilchen eine pH-Wert-Stabilisierung (Pufferung) in späteren Anwendungen, insbesondere in solchen Polymeren, die Säuregruppen enthalten oder unter bestimmten Bedingungen Säuren freisetzen können. Hierzu gehören beispielsweise Polyvinylchlorid und Polymilchsäure. Ferner können durch die erfindungsgemäßen Verbundteilchen ggf. andere, teurere Werkstoffe ersetzt werden, um so eine Verbilligung des Endprodukts zu erreichen.

Die Weiterverarbeitung der erfindungsgemäßen Verbundteilchen kann auf vergleichbar einfache Art und Weise erfolgen, da nach der erfindungsgemäßen Lösung nur eine Komponente (die Verbundteilchen) und nicht mehr zwei Komponenten (Calciumsalz und Polymer) zu verarbeiten sind. Dispergierprobleme sind aufgrund der festen Verbindung zwischen dem Polymer und dem Calciumcarbonat nicht zu beobachten.

Weiterhin kann über die Wahl der Anteile und der Größe der jeweiligen Einzelkomponenten die Mikrostruktur, die Porosität und die Permeabilität der Verbundteilchen gezielt gesteuert werden. Die durchgehend steuerbare Permeabilität, die durchgehend steuerbare Porosität und die durchgehend steuerbare Isotropie der Verbundteilchen kann wiederum genutzt werden, um die Endstruktur der resultierenden Bauteile, insbesondere ihre Mikrostruktur, ihre Porosität und ihre Permeabilität, gezielt zu steuern.

Ein Zusatz von weiteren Verarbeitungshilfsmitteln, insbesondere von speziellen Lösungsmitteln, ist bei der Verarbeitung der erfindungsgemäßen Verbundteilchen in der Regel nicht erforderlich. Dies erweitert die möglichen Anwendungsgebiete der erfindungsgemäßen Verbundteilchen insbesondere im Pharma- und im Nahrungsmittelsektor.

Die erfindungsgemäßen Verbundteilchen können als solche direkt eingesetzt werden. Aufgrund ihres hervorragenden Eigenschaftsprofils eignen sich die erfindungsgemäßen Verbundteilchen jedoch insbesondere als Additiv, besonders bevorzugt als Polymeradditiv, als Zusatzstoff oder Ausgangsmaterial für die Herstellung von Bauteilen, für Anwendungen in der Medizintechnik und/oder in der Mikrotechnik und/oder für die Herstellung von geschäumten Gegenständen. Besonders bevorzugte medizintechnische Anwendungen schließen vorzugsweise resorbierbare Implantate ein. Besonders zweckmäßige Anwendungsgebiete umfassen spritzgegossene Schrauben, gepresste Platten, insbesondere schmelzgepresste Platten, geschäumte Implantate sowie rieselfähige Pulver für selektive Fertigungsverfahren, wobei im letzteren Fall die Gesamtteilchengröße vorzugsweise kleiner 3 mm und vorzugsweise größer 0,5 µm ist.

Als Polymeradditiv werden die erfindungsgemäßen Verbundteilchen vorzugsweise mindestens einem Polymer, insbesondere einem thermoplastischen Polymer, als Matrixpolymer zugesetzt. Besonders bevorzugt werden hier die Polymere, die auch als Komponente der erfindungsgemäßen Verbundteilchen verwendet werden können. Um Wiederholungen zu vermeiden, wird daher auf die obigen Ausführungen verwiesen, insbesondere hinsichtlich der bevorzugten Formen des Polymers. Ganz besonders bevorzugte Matrixpolymere schließen Polyvinylchlorid (PVC), Polyurethan (PU), Silikon, Polypropylen (PP), Polyethylen (PE) und Polymilchsäure (PLA) ein.

Im Rahmen der vorliegenden Erfindung sind das Matrixpolymer und das Polymer der Verbundteilchen vorzugsweise bei der Anwendungstemperatur miteinander mischbar, besonders bevorzugt chemisch identisch.

Besonders bevorzugte Zusammensetzungen enthalten 40,0 Gew.-% bis 99,9 Gew.-% mindestens eines Matrixpolymers und 0,1 Gew.-% bis 50,0 Gew.-% mindestens eines erfindungsgemäßen Verbundteilchens.

Die Herstellung der Zusammensetzung kann auf an sich bekannte Weise durch Mischen der Komponenten erfolgen.

Die Zusammensetzung kann dann in üblicherweise weiterverarbeitet, insbesondere granuliert, gemahlen, extrudiert, spritzgegossen oder auch geschäumt werden.

Weiterhin können die erfindungsgemäßen Verbundteilchen direkt, d. h. ohne Zusatz von zusätzlichen Polymeren, weiterverarbeitet und/oder verwendet werden.

Die Vorteile der erfindungsgemäßen Verbundteilchen sind dabei insbesondere beim Granulieren, Mahlen, Extrudieren, Spritzgießen, Schmelzpressen und/oder Schäumen der Verbundteilchen zu beobachten.

Im Rahmen der vorliegenden Beschreibung erfolgt die Herstellung von Polymerschäumen vorzugsweise durch die Erzeugung oder Einbringung einer gasförmigen Phase in eine Zusammensetzung, umfassend die erfindungsgemäßen Verbundteilchen und ggf. mindestens ein Matrixpolymer. Ziel ist es dabei, das Gas möglichst gleichmäßig in der Zusammensetzung zu verteilen, um eine gleichmäßige und homogene Schaumstruktur zu erreichen. Die Einbringung des Gases kann auf verschiedene Weise erfolgen.

Bevorzugt erfolgt die Erzeugung der Gasphase durch Zugabe eines Treibmittels. Als Treibmittel werden Substanzen bezeichnet, die durch chemische Reaktionen (chemische Treibmittel) oder durch Phasenübergang (physikalische Treibmittel) Gase freisetzen. Bei der Schaumextrusion oder beim Schaumspritzguss wird das chemische Treibmittel in Form eines Masterbatches der Zusammensetzung beigemengt oder physikalisches Treibmittel direkt in die Schmelze der Zusammensetzung unter Druck injiziert. Die Injektion wird als Direktbegasung bezeichnet und findet insbesondere bei der Verarbeitung von thermoplastischen Polymeren Einsatz.

Insbesondere für diese Anwendung ist es von Vorteil, wenn die erfindungsgemäßen Verbundteilchen eine Struktur aufweisen, die es erlaubt, dass eine fluide Substanz, bevorzugt das Treibmittel, in das Polymer eindringen kann und vorzugsweise dieses zumindest teilweise lösen kann. Bei Verbundteilchen mit einem Kern, umfassend mindestens ein vorzugsweise thermoplastisches Polymer, und eine Hülle, umfassend mindestens ein gefälltes Calciumcarbonat, ist die Hülle für die fluide Substanz, insbesondere für das Treibmittel, zumindest teilweise permeabel. Dies wird vorzugsweise dadurch erreicht, dass mindestens 0,1 %, bevorzugt mindestens 0,5 %, insbesondere 1,0 % bis 5 %, der Kernoberfläche nicht mit gefälltem Calciumcarbonat beschichtet sind. Verstärkt wird dieser Effekt vorzugsweise durch die Lücken zwischen einzelnen gefällten Calciumcarbonat-Teilchen, die vorzugsweise vorliegen und zur Ausbildung von entsprechenden Mikrokanälen für die fluide Substanz, insbesondere für das Treibmittel, führen.

Im Rahmen einer besonders bevorzugten Ausführungsform der vorliegenden Erfindung werden die Verbundteilchen in Anlehnung an die in der Publikation M. Avella, S. Cosco, M. L. Di Lorenzo, E. Di Pace, M. E. Errico Influence of CaCO3 Nanoparticles Shape on Thermal and Crystallization Behavior of Isotactic Polypropylene based Nanocomposites Journal of Thermal Analysis and Calorimetry, Vol. 80 (2005) 131-136 beschriebenen Vorgehensweise aufgeschäumt. Dabei werden die erfindungsgemäßen Verbundteilchen, die vorzugsweise mindestens ein resorbierbares Polymer, vorzugsweise mindestens einen resorbierbaren Polyester, insbesondere Polymilchsäure, umfassen, vorzugsweise mit CO₂, bevorzugt unter hohem Druck, zweckmäßigerweise bei Raumtemperatur, begast. Dabei wird CO₂ vom Polymer aufgenommen und dessen Glasübergangstemperatur vorzugsweise gesenkt. Bei Druckreduktion kommt es zum Aufschäumen der Verbundteilchen und vorzugsweise zum exaktem Ausfließen der Form, bevorzugt einer Teflonform. Das Verfahren ermöglicht damit insbesondere das Einbringen thermolabiler Substanzen, vor allem von Antibiotika und osteoinduktiver Proteinen.

Im Folgenden wird die vorliegende Erfindung durch mehrere Beispiele weiter veranschaulicht, ohne dass hierdurch eine Beschränkung des Erfindungsgedankens erfolgen soll.

### Charakterisierung

Die Eigenschaften der mikrostruktierten Verbundteilchen-Teilchen wurden wie folgt bestimmt.

### Elektronenmikroskop

Die rasterelektronischen Aufnahmen wurden mit einem

Hochspannungselektronenmikroskop (Zeiss, DSM 962) bei 15 kV durchgeführt. Die Proben wurden mit einer Gold-Palladiumschicht besprüht.

### Thermogravimetrie (TGA)

Die Thermogravimetrie wurde mit einem PerkinElmer STA 6000 unter Stickstoff (Stickstoff-Durchflußmenge: 20 ml/min) im Bereich von 40°C bis 1000°C bei einer Heizrate von 10°C/min durchgeführt.

### Beispiel 1

Mikrostrukturierte Verbundteilchen aus amorphem Calciumcarbonat und einem amorphen Polylactid (PLA) wurden in Anlehnung an das in JP 62083029 A beschriebene Verfahren unter Verwendung des Geräts NHS-0 hergestellt. Es wurde mit 12°C kaltem Wasser gekühlt. Als Mutterpartikel wurde ein Polylactid-Granulat (mittlere Teilchengröße 3 mm) und als die Babypartikel amorphes Calciumcarbonat-Pulver (DSACC; mittlere Teilchengröße 1 µm) verwendet.

16 g Polylactid-Granulat wurden mit 4 g CaCO₃-Pulver vermengt und bei 5.000 U/min befüllt. Die Rotorgeschwindigkeit des Aggregats wurde auf 16.000 U/min (100 m/s) eingestellt und die zudosierten Materialien für 1 min verarbeitet. Diese Prozedur wurde mit den gleichen Materialmengen und Maschineneinstellungen wiederholt. Es wurden insgesamt 38 g strukturierte Verbundteilchen gewonnen.

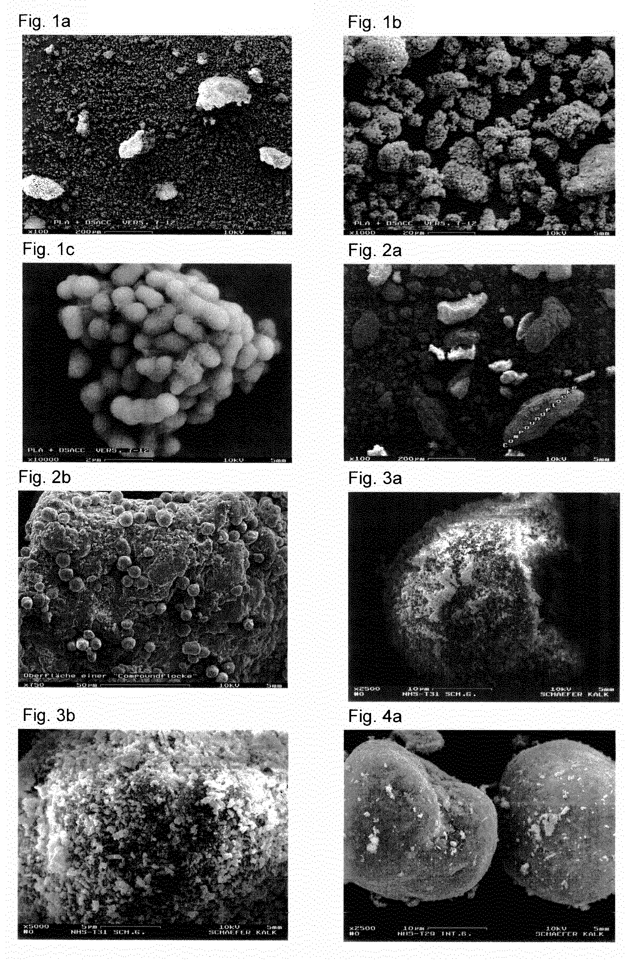

Die REM-Analyse zeigte, dass die PLA-Oberfläche weitgehend mit den kugelförmigen DSACC-Teilchen bedeckt ist (siehe Fig. 1a, 1b, 1c).

### Beispiel 2

Mikrostrukturierte Verbundteilchen aus Calciumcarbonat-Kugeln (Sphärolithen; SPH) und einem amorphen Polylactid (PLA) wurden mit dem Einsatz von NHS-0 wie im Beispiel 1 beschrieben hergestellt. Als Mutterpartikel wurde gleiches Polylactid-Granulat, wie im Beispiel 1 beschrieben, und als die Babypartikel Calciumcarbonat-Kugeln (Sphärolithen) mit einem mittleren Teilchendurchmesser von 7 µm verwendet.

16 g Polylactid-Granulat wurden mit 4 g CaCO₃-Pulver vermengt und bei 5.000 U/min befüllt. Die Rotorgeschwindigkeit des Aggregats wurde auf 16.000 U/min (100 m/s) eingestellt und die zudosierten Materialien für 1 min verarbeitet. Es wurden insgesamt 5 Wiederholungen mit den gleichen Materialmengen und Maschineneinstellungen durchgeführt. Es wurden insgesamt 85 g strukturierte Verbundteilchen gewonnen.

Die REM-Analyse der gewonnenen strukturierten Verbundteilchen ist auf den folgenden REM-Aufnahmen dargestellt. Die PLA-Oberfläche ist nur zum Teil mit den Calciumcarbonat-Kugeln (Sphärolithen) bedeckt (siehe Fig. 2a, 2b).

### Beispiel 3

Mikrostrukturierte Verbundteilchen aus einem Calciumcarbonat mit gemischter Partikelform (Skalenoeder und Nadeln; Schaefer Precarb® 400) und einem Feinpulver auf der Basis von Polyamid-12 (PA12) wurden mit dem Einsatz von NHS-1 hergestellt. Es wurde mit 12°C kaltem Wasser gekühlt. Als Mutterpartikel wurde PA12 (mittlere Teilchengröße 50 µm) und als die Babypartikel Calciumcarbonat Schaefer Precarb® 400 (mittlere Teilchengröße 0,7 µm) verwendet.

85 g PA12-Pulver wurden mit 15 g CaCO₃-Pulver Schaefer Precarb® 400 vermengt und bei einer Rotorgeschwindigkeit des Aggregats von 4.000 U/min (50 m/s) befüllt. Die zudosierten Materialien wurden für 1 min verarbeitet. Es wurden insgesamt 8 Wiederholungen mit den gleichen Materialmengen und Maschineneinstellungen durchgeführt. Es wurden insgesamt ca. 760 g strukturierte Verbundteilchen gewonnen.

Die REM-Analyse der gewonnenen strukturierten Verbundteilchen ist in Fig. 3a, 3b dargestellt.

Der mittels Thermogravimetrie ermittelte CaCO₃-Gehalt betrug 14,4 % PCC.

Die Partikelgroßenverteilung der gewonnen strukturierten Verbundteilchen wurde mittels Laserbeugung (Sympatec, Helos) bestimmt (d₅₀ = 48 µm).

### Beispiel 4

Mikrostrukturierte Verbundteilchen aus einem Calciumcarbonat mit gemischter Partikelform (Skalenoeder und Nadeln; Schaefer Precarb® 400) und einem Feinpulver auf der Basis von Polyamid-12 (PA12) wurden mit dem Einsatz von NHS-1 hergestellt. Es wurde mit 12°C kaltem Wasser gekühlt. Als Mutterpartikel wurde PA12 (mittlere Teilchengröße 50 µm) und als die Babypartikel Calciumcarbonat Schaefer Precarb® 400 (mittlere Teilchengröße 0,7 µm) verwendet.

85 g PA12-Pulver wurden mit 15 g CaCO₃-Pulver Schaefer Precarb® 400 vermengt und bei einer Rotorgeschwindigkeit des Aggregats von 8.000 U/min (100 m/s) befüllt. Die zudosierten Materialien wurden für 3 min verarbeitet. Es wurden insgesamt 2 Wiederholungen mit den gleichen Materialmengen und Maschineneinstellungen durchgeführt. Es wurden insgesamt ca. 196 g strukturierte Verbundteilchen gewonnen.

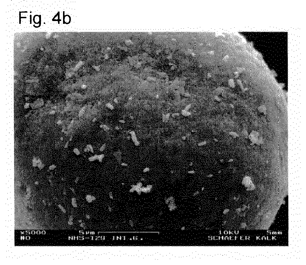

Die REM-Analyse der gewonnenen strukturierten Verbundteilchen ist in Fig. 4a, 4b dargestellt.

Der mittels Thermogravimetrie ermittelte CaCO₃-Gehalt betrug 14,1 % PCC.

Die Partikelgroßenverteilung der gewonnen strukturierten Verbundteilchen wurde mittels Laserbeugung (Sympatec, Helos) bestimmt (d₅₀ = 51 µm).

## Patentansprüche

1. Mikrostrukturierte Verbundteilchen, erhältlich durch ein Verfahren, bei welchem man große Teilchen mit kleinen Teilchen verbindet, wobei
- die großen Teilchen einen mittleren Teilchendurchmesser im Bereich von 0,1 µm bis 10 mm aufweisen,
- der mittlere Teilchendurchmesser der kleinen Teilchen höchstens 1/10 des mittleren Teilchendurchmessers der großen Teilchen ist,
- die großen Teilchen mindestens ein Polymer umfassen,
- die kleinen Teilchen Calciumcarbonat umfassen,
- die kleinen Teilchen auf der Oberfläche der großen Teilchen angeordnet und/oder inhomogen innerhalb der großen Teilchen verteilt sind,
**dadurch gekennzeichnet, dass** die kleinen Teilchen gefällte Calciumcarbonat-Teilchen mit einer mittleren Teilchengröße im Bereich von 0,01 µm bis 1,0 mm umfassen.

2. Verbundteilchen nach Anspruch 1, **dadurch gekennzeichnet, dass** das Calciumcarbonat einen Aspekt Ratio kleiner 5 aufweist.

3. Verbundteilchen nach Anspruch 1 oder 2, **dadurch gekennzeichnet, dass** das Calziumcarbonat kugelförmiges Calciumcarbonat umfasst.

4. Verbundteilchen nach mindestens einem der vorangehenden Ansprüche, **dadurch gekennzeichnet, dass** das Calciumcarbonat stabilisierte Calciumcarbonat-Teilchen umfasst, wobei die Calciumcarbonat-Teilchen mindestens eine Substanz umfassen, die eine Molmasse größer 100 g/mol aufweist und der Formel R-Xₙ genügt, wobei der Rest R für einen mindestens ein Kohlenstoffatom umfassenden Rest steht, der Rest X für eine Gruppe steht, die mindestens ein Sauerstoffatom sowie mindestens ein Kohlenstoffatom, Schwefelatom, Phosphoratom und/oder Stickstoffatom umfasst und n eine vorzugsweise ganze Zahl im Bereich von 1 bis 20 darstellt.

5. Verbundteilchen nach Anspruch 1, **dadurch gekennzeichnet, dass** die großen Teilchen mindestens ein thermoplastisches Polymer umfassen.

6. Verbundteilchen nach Anspruch 1, **dadurch gekennzeichnet, dass** die großen Teilchen mindestens ein resorbierbares Polymer umfassen.

7. Verbundteilchen nach mindestens einem der vorangehenden Ansprüche, **dadurch gekennzeichnet, dass** die großen Teilchen Poly-D-, Poly-L- und/oder Poly-D,L-milchsäure umfassen.

8. Verbundteilchen nach mindestens einem der vorangehenden Ansprüche, **dadurch gekennzeichnet, dass** die großen Teilchen mindestens ein Polyamid umfassen.

9. Verbundteilchen nach mindestens einem der vorangehenden Ansprüche, **dadurch gekennzeichnet, dass** die kleinen Teilchen mindestens ein Calciumphosphat umfassen.

10. Verbundteilchen nach Anspruch 9, **dadurch gekennzeichnet, dass** die kleinen Teilchen Ca₃(PO₄)₂, CaHPO₄, Ca(H₂PO₄)₂ und/oder Ca₅(PO₄)₃(OH) umfassen.

11. Verbundteilchen nach mindestens einem der vorangehenden Ansprüche, **dadurch gekennzeichnet, dass** die Verbundteilchen einen Kern und eine Hülle umfassen, wobei der Kern einen mittleren Durchmesser im Bereich von 0,5 µm bis 2,0 mm und die Hülle eine mittlere Dicke von höchstens 20 %, bezogen auf den Kerndurchmesser, aufweist.

12. Verbundteilchen nach mindestens einem der vorangehenden Ansprüche, **dadurch gekennzeichnet, dass** der Gewichtsanteil des Calciumcarbonates, bezogen auf das Gesamtgewicht der Verbundteilchen, mindestens 0,1 Gew.-% beträgt.

13. Verwendung von Verbundteilchen nach mindestens einem der vorangehenden Ansprüche als Additiv, insbesondere als Polymeradditiv, als Zusatzsatzstoff oder Ausgangsmaterial für die Herstellung von Bauteilen, für Anwendungen in der Medizintechnik und/oder in der Mikrotechnik und/oder für die Herstellung von geschäumten Gegenständen.

## Claims

1. Microstructured composite particles obtainable by a process comprising combining large particles with small particles, wherein
- the large particles have an average particle diameter in the range from 0.1 µm to 10 mm,
- the average particle diameter of the small particles is not more than 1/10 of the average particle diameter of the large particles,
- the large particles comprise at least one polymer,
- the small particles comprise calcium carbonate,
- the small particles form an arrangement on the surface of the large particles and/or an inhomogeneous distribution within the large particles,
wherein the small particles comprise precipitated particles of calcium carbonate which have an average particle size in the range from 0.01 µm to 1.0 mm.

2. The composite particles according to claim 1, wherein the calcium salt has an aspect ratio below 5.

3. The composite particles according to claim 1 or 2, wherein the calcium carbonate comprises sphere-shaped calcium carbonate.

4. The composite particles according to one or more of the preceding claims, wherein the calcium carbonate comprises stabilized particles of calcium carbonate, wherein the particles of calcium carbonate comprise at least one substance having a molar mass above 100 g/mol and satisfying the formula R-Xₙ, where the radical R represents a radical comprising at least one carbon atom, the radical X represents a group comprising at least one oxygen atom and also at least one carbon atom, sulfur atom, phosphorus atom and/or nitrogen atom, and n represents a preferably whole number in the range from 1 to 20.

5. The composite particles according to claim 1, wherein the large particles comprise at least one thermoplastic polymer.

6. The composite particles according to claim 1, wherein the large particles comprise at least one reabsorbable polymer.

7. The composite particles according to one or more of the preceding claims, wherein the large particles comprise poly-D-, poly-L- and/or poly-D,L-lactic acid.

8. The composite particles according to one or more of the preceding claims, wherein the large particles comprise at least one polyamide.

9. The composite particles according to one or more of the preceding claims, wherein the small particles comprise at least one calcium phosphate.

10. The composite particles according to claim 9, wherein the small particles comprise Ca₃(PO₄)₂, CaHPO₄, Ca(H₂PO₄)₂ and/or Ca₅(PO₄)₃(OH).

11. The composite particles according to one or more of the preceding claims, wherein the composite particles comprise a core and a sheath, wherein the core has an average diameter in the range from 0.5 µm to 2.0 mm and the sheath has an average thickness of not more than 20%, based on the core diameter.

12. The composite particles according to one or more of the preceding claims, wherein the weight fraction of calcium salt, based on the overall weight of the composite particles, is not less than 0.1 wt%.

13. Use of composite particles according to one or more of the preceding claims as an additive, especially as a polymer additive, as an adjuvant or as a starting material for the production of structural components, for applications in biomedical engineering and/or in microtechnology and/or for the production of foamed articles.

## Revendications

1. Particules composites microstructurées pouvant être obtenues par un procédé dans lequel on lie de grandes particules avec de petites particules, où
- les grandes particules présentent un diamètre de particule moyen dans le domaine de 0,1 µm à 10 mm,
- le diamètre de particule moyen des petites particules est au plus 1/10 du diamètre de particule moyen des grandes particules,
- les grandes particules comprennent au moins un polymère,
- les petites particules comprennent du carbonate de calcium,
- les petites particules sont disposées sur la surface des grandes particules et/ou réparties de manière non homogène à l'intérieur des grandes particules,
**caractérisées en ce que** les petites particules comprennent des particules de carbonate de calcium précipitées ayant une dimension de particule moyenne dans le domaine de 0,01 µm à 1,0 mm.

2. Particules composites selon la revendication 1, **caractérisées en ce que** le carbonate de calcium présente un rapport d'aspect inférieur à 5.

3. Particules composites selon la revendication 1 ou 2, **caractérisées en ce que** le carbonate de calcium comprend du carbonate de calcium sphérique.

4. Particules composites selon au moins l'une des revendications précédentes, **caractérisées en ce que** le carbonate de calcium comprend des particules de carbonate de calcium stabilisées, où les particules de carbonate de calcium comprennent au moins une substance qui présente une masse molaire supérieure à 100 g/mole et répond à la formule R-Xₙ où le groupement R représente un groupement comprenant au moins un atome de carbone, le groupement X représente un groupe qui comprend au moins un atome d'oxygène ainsi qu'au moins un atome de carbone, un atome de soufre, un atome de phosphore et/ou un atome d'azote et n représente un nombre de préférence entier dans le domaine de 1 à 20.

5. Particules composites selon la revendication 1, **caractérisées en ce que** les grandes particules comprennent au moins un polymère thermoplastique.

6. Particules composites selon la revendication 1, **caractérisées en ce que** les grandes particules comprennent au moins un polymère résorbable.

7. Particules composites selon au moins l'une des revendications précédentes, **caractérisées en ce que** les grandes particules comprennent du poly-D-, du poly-L- et/ou du poly-D,L-acide lactique.

8. Particules composites selon au moins l'une des revendications précédentes, **caractérisées en ce que** les grandes particules comprennent au moins un polyamide.

9. Particules composites selon au moins l'une des revendications précédentes, **caractérisées en ce que** les petites particules comprennent au moins un phosphate de calcium.

10. Particules composites selon la revendication 9, **caractérisées en ce que** les petites particules comprennent Ca₃(PO₄)₂, CaHPO₄, Ca(H₂PO₄)₂ et/ou Ca₅(PO₄)₃(OH).

11. Particules composites selon au moins l'une des revendications précédentes, **caractérisées en ce que** les particules composites comprennent un noyau et une enveloppe, où le noyau présente un diamètre moyen dans le domaine de 0,5 µm à 2,0 mm et l'enveloppe présente une épaisseur moyenne d'au plus 20 %, par rapport au diamètre du noyau.

12. Particules composites selon au moins l'une des revendications précédentes, **caractérisées en ce que** la fraction en poids du carbonate de calcium, par rapport au poids total des particules composites, est d'au moins 0,1 % en poids.

13. Utilisation de particules composites selon au moins l'une des revendications précédentes comme additif, en particulier comme additif de polymère, comme produit ajouté ou comme matière première pour la fabrication d'éléments constitutifs, pour des applications en technique médicale et/ou en microtechnique et/ou pour la fabrication d'objets expansés.
